# EUROPEAN PATENT SPECIFICATION

(11) **EP 2 209 488 B1**
(45) Date of publication and mention of the grant of the patent: **16.07.2014**
(21) Application number: 08843418.8
(22) Date of filing: 03.11.2008
(51) Int. Cl.: A61K 38/18, A61P 25/22, A61P 25/24

(54) **FGF9-RELATED METHODS FOR TREATING ANXIETY**
FGF9-ASSOZIIERTE VERFAHREN ZUR BEHANDLUNG VON ANGSTZUSTÄNDEN
PROCÉDÉS ASSOCIÉS À FGF9 PERMETTANT DE TRAITER L'ANXIÉTÉ

(30) Priority: 02.11.2007 US 985146 P
(43) Date of publication of application: 28.07.2010
(73) Proprietor: The Board of Trustees of the Leland Stanford Junior University, Palo Alto, CA 94306-1106 (US)
(72) Inventor: AKIL, Huda, Ann Arbor, MI 48105 (US); WATSON, Stanley, Ann Arbor, MI 48105 (US); TURNER, Cortney, Anne Arbor, MI 48108 (US)
(74) Representative: Campbell, Patrick John Henry
(86) International application number: PCT/US2008/082279
(87) International publication number: WO 2009/059316

(56) References cited:
- WO-A2-2007/059064
- US-A1- 2002 169 102
- US-A1- 2007 213 401
- GOMEZ-PINILLA ET AL.: 'Diazepam induces FGF-2 mRNA in the hippocampus and striatum.' BRAIN RESEARCH BULLETIN vol. 53, no. 3, 2000, pages 283 - 289, XP002462753
- EVANS ET AL. PNAS vol. 43, no. 101, 26 October 2004, pages 15506 - 15511, XP002552309

### SEQUENCE LISTING

<110> The Board of Trustees of The Leland Stanford Junior University
<120> FGF9-Related Methods for Treating Anxiety
<130> N.110917
<140> EP 08843418.8
   <141> 2008-11-03
<150> US 60/985,146
   <151> 2007-11-02
<160> 38
<170> FastSEQ for Windows Version 4.0
<210> 1
   <211> 21
   <212> DNA
   <213> Artificial Sequence
<220>
   <223> synthetic real time RT-PCR fibroblast growth factor receptor 2 (FGFR2) exon 2 amplicon forward primer R2 F
<400> 1
   gccgtgatca gttggactaa g 21
<210> 2
   <211> 21
   <212> DNA
   <213> Artificial Sequence
<220>
   <223> synthetic real time RT-PCR fibroblast growth factor receptor 2 (FGFR2) exon 2 amplicon reverse primer R2 R
<400> 2
   tgtggcacct tttatctgga g 21
<210> 3
   <211> 21
   <212> DNA
   <213> Artificial Sequence
<220>
   <223> synthetic real time RT-PCR fibroblast growth factor receptor 2 (FGFR2) exon 5 amplicon forward primer R2 F
<400> 3
   tatggaaagt gtggtcccat c 21
<210> 4
   <211> 21
   <212> DNA
   <213> Artificial Sequence
<220>
   <223> synthetic real time RT-PCR fibroblast growth factor receptor 2 (FGFR2) exon 5 amplicon reverse primer R2 R
<400> 4
   acatcaaggt ggtaggtgtg g 21
<210> 5
   <211> 21
   <212> DNA
   <213> Artificial Sequence
<220>
   <223> synthetic real time RT-PCR fibroblast growth factor receptor 2 (FGFR2) exon 6 amplicon forward primer R2 F
<400> 5
   ggaggggacg tagaatttgt c 21
<210> 6
   <211> 21
   <212> DNA
   <213> Artificial Sequence
<220>
   <223> synthetic real time RT-PCR fibroblast growth factor receptor 2 (FGFR2) exon 6 amplicon reverse primer R2 R
<400> 6
   cttcaggacc ttgaggtagg g 21
<210> 7
   <211> 21
   <212> DNA
   <213> Artificial Sequence
<220>
   <223> synthetic real time RT-PCR fibroblast growth factor receptor 2 (FGFR2) exon IIIb amplicon forward primer R2 F
<400> 7
   ggggataaat agctccaatg c 21
<210> 8
   <211> 24
   <212> DNA
   <213> Artificial Sequence
<220>
   <223> synthetic real time RT-PCR fibroblast growth factor receptor 2 (FGFR2) exon IIIb amplicon reverse primer R2 R
<400> 8
   catatatatt ccccagcatc catc 24
<210> 9
   <211> 21
   <212> DNA
   <213> Artificial Sequence
<220>
   <223> synthetic real time RT-PCR fibroblast growth factor receptor 2 (FGFR2) exon IIIc amplicon forward primer R2 F
<400> 9
   acaccacgga caaagaaatt g 21
<210> 10
   <211> 21
   <212> DNA
   <213> Artificial Sequence
<220>
   <223> synthetic real time RT-PCR fibroblast growth factor receptor 2 (FGFR2) exon IIIc amplicon reverse primer R2 R
<400> 10
   atagaattac ccgccaagca c 21
<210> 11
   <211> 22
   <212> DNA
   <213> Artificial Sequence
<220>
   <223> synthetic real time RT-PCR fibroblast growth factor receptor 2 (FGFR2) exon 8 amplicon forward primer R2 F
<400> 11
   gatcacagct tccccagatt ac 22
<210> 12
   <211> 21
   <212> DNA
   <213> Artificial Sequence
<220>
   <223> synthetic real time RT-PCR fibroblast growth factor receptor 2 (FGFR2) exon 8 amplicon reverse primer R2 R
<400> 12
   tcttggtcgt ggtcttcatt c 21
<210> 13
   <211> 22
   <212> DNA
   <213> Artificial Sequence
<220>
   <223> synthetic real time RT-PCR fibroblast growth factor receptor 2 (FGFR2) exon 11 amplicon forward primer R2 F
<400> 13
   agagaaggac ctgtctgacc tg 22
<210> 14
   <211> 21
   <212> DNA
   <213> Artificial Sequence
<220>
   <223> synthetic real time RT-PCR fibroblast growth factor receptor 2 (FGFR2) exon 11 amplicon reverse primer R2 R
<400> 14
   cccaggaggt tgatgatgtt c 21
<210> 15
   <211> 21
   <212> DNA
   <213> Artificial Sequence
<220>
   <223> synthetic real time RT-PCR fibroblast growth factor receptor 2 (FGFR2) exon 15 amplicon forward primer R2 F
<400> 15
   gtccttcggg gtgttaatgt g 21
<210> 16
   <211> 21
   <212> DNA
   <213> Artificial Sequence
<220>
   <223> synthetic real time RT-PCR fibroblast growth factor receptor 2 (FGFR2) exon 2 amplicon forward primer R2 F
<400> 16
   agttcattgg tgcagttggt g 21
<210> 17
   <211> 21
   <212> DNA
   <213> Artificial Sequence
<220>
   <223> synthetic real time RT-PCR fibroblast growth factor receptor 3 (FGFR3) exon 2 amplicon forward primer R3 F
<400> 17
   agaggcttca agtgctaaac g 21
<210> 18
   <211> 20
   <212> DNA
   <213> Artificial Sequence
<220>
   <223> synthetic real time RT-PCR fibroblast growth factor receptor 3 (FGFR3) exon 2 amplicon reverse primer R3 R
<400> 18
   gcacactaaa gtggcacagc 20
<210> 19
   <211> 20
   <212> DNA
   <213> Artificial Sequence
<220>
   <223> synthetic real time RT-PCR fibroblast growth factor receptor 3 (FGFR3) exon 5 amplicon forward primer R3 F
<400> 19
   tggagcttgg tcatggaaag 20
<210> 20
   <211> 20
   <212> DNA
   <213> Artificial Sequence
<220>
   <223> synthetic real time RT-PCR fibroblast growth factor receptor 3 (FGFR3) exon 5 amplicon reverse primer R3 R
<400> 20
   ggatgctgcc aaacttgttc 20
<210> 21
   <211> 18
   <212> DNA
   <213> Artificial Sequence
<220>
   <223> synthetic real time RT-PCR fibroblast growth factor receptor 3 (FGFR3) exon 6 amplicon forward primer R3 F
<400> 21
   ccaaccagac agccgttc 18
<210> 22
   <211> 19
   <212> DNA
   <213> Artificial Sequence
<220>
   <223> synthetic real time RT-PCR fibroblast growth factor receptor 3 (FGFR3) exon 6 amplicon reverse primer R3 R
<400> 22
   cattcacctc cacgtgctt 19
<210> 23
   <211> 21
   <212> DNA
   <213> Artificial Sequence
<220>
   <223> synthetic real time RT-PCR fibroblast growth factor receptor 3 (FGFR3) exon IIIb amplicon forward primer R3 F
<400> 23
   cctggatcag tgagaatgtg g 21
<210> 24
   <211> 20
   <212> DNA
   <213> Artificial Sequence
<220>
   <223> synthetic real time RT-PCR fibroblast growth factor receptor 3 (FGFR3) exon IIIb amplicon reverse primer R3 R
<400> 24
   aaattggtgg ctcgacagag 20
<210> 25
   <211> 20
   <212> DNA
   <213> Artificial Sequence
<220>
   <223> synthetic real time RT-PCR fibroblast growth factor receptor 3 (FGFR3) exon IIIc amplicon forward primer R3 F
<400> 25
   tgtccttgca caatgtcacc 20
<210> 26
   <211> 20
   <212> DNA
   <213> Artificial Sequence
<220>
   <223> synthetic real time RT-PCR fibroblast growth factor receptor 3 (FGFR3) exon IIIc amplicon reverse primer R3 R
<400> 26
   acgcagagtg atgggaaaac 20
<210> 27
   <211> 20
   <212> DNA
   <213> Artificial Sequence
<220>
   <223> synthetic real time RT-PCR fibroblast growth factor receptor 3 (FGFR3) exon 8 amplicon forward primer R3 F
<400> 27
   ggaggagctg atggaagttg 20
<210> 28
   <211> 20
   <212> DNA
   <213> Artificial Sequence
<220>
   <223> synthetic real time RT-PCR fibroblast growth factor receptor 3 (FGFR3) exon 8 amplicon reverse primer R3 R
<400> 28
   ccaccaggat gaagaggaag 20
<210> 29
   <211> 20
   <212> DNA
   <213> Artificial Sequence
<220>
   <223> synthetic real time RT-PCR fibroblast growth factor receptor 3 (FGFR3) exon 11 amplicon forward primer R3 F
<400> 29
   atgccactga caaggacctg 20
<210> 30
   <211> 20
   <212> DNA
   <213> Artificial Sequence
<220>
   <223> synthetic real time RT-PCR fibroblast growth factor receptor 3 (FGFR3) exon 11 amplicon reverse primer R3 R
<400> 30
   cccccaacag gttaatgatg 20
<210> 31
   <211> 20
   <212> DNA
   <213> Artificial Sequence
<220>
   <223> synthetic real time RT-PCR fibroblast growth factor receptor 3 (FGFR3) exon 15 amplicon forward primer R3 F
<400> 31
   tcctttggtg tcctcctctg 20
<210> 32
   <211> 18
   <212> DNA
   <213> Artificial Sequence
<220>
   <223> synthetic real time RT-PCR fibroblast growth factor receptor 3 (FGFR3) exon 15 amplicon reverse primer R3 R
<400> 32
   cagttggctg gcttgtcc 18
<210> 33
   <211> 200
   <212> PRT
   <213> Artificial Sequence
<220>
   <223> synthetic poly Gly flexible linker
<220>
   <221> MOD RES
   <222> (6)...(200)
   <223> Gly at positions 6-200 may be present or absent
<400> 33
   Gly Gly Gly Gly Gly Gly Gly Gly Gly Gly Gly Gly Gly Gly Gly Gly
   1 5 10 15
   Gly Gly Gly Gly Gly Gly Gly Gly Gly Gly Gly Gly Gly Gly Gly Gly
   20 25 30
   Gly Gly Gly Gly Gly Gly Gly Gly Gly Gly Gly Gly Gly Gly Gly Gly
   35 40 45
   Gly Gly Gly Gly Gly Gly Gly Gly Gly Gly Gly Gly Gly Gly Gly Gly
   50 55 60
   Gly Gly Gly Gly Gly Gly Gly Gly Gly Gly Gly Gly Gly Gly Gly Gly
   65 70 75 80
   Gly Gly Gly Gly Gly Gly Gly Gly Gly Gly Gly Gly Gly Gly Gly Gly
   85 90 95
   Gly Gly Gly Gly Gly Gly Gly Gly Gly Gly Gly Gly Gly Gly Gly Gly
   100 105 110
   Gly Gly Gly Gly Gly Gly Gly Gly Gly Gly Gly Gly Gly Gly Gly Gly
   115 120 125
   Gly Gly Gly Gly Gly Gly Gly Gly Gly Gly Gly Gly Gly Gly Gly Gly
   130 135 140
   Gly Gly Gly Gly Gly Gly Gly Gly Gly Gly Gly Gly Gly Gly Gly Gly
   145 150 155 160
   Gly Gly Gly Gly Gly Gly Gly Gly Gly Gly Gly Gly Gly Gly Gly Gly
   165 170 175
   Gly Gly Gly Gly Gly Gly Gly Gly Gly Gly Gly Gly Gly Gly Gly Gly
   180 185 190
   Gly Gly Gly Gly Gly Gly Gly Gly
   195 200
<210> 34
   <211> 15
   <212> PRT
   <213> Artificial Sequence
<220>
<210> 35
   <211> 9
   <212> PRT
   <213> Artificial Sequence
<220>
   <223> synthetic FGF system peptide inhibitor, FGFR peptide inhibitor
<400> 35
   His Phe Lys Asp Pro Lys Arg Leu Tyr
    1               5
<210> 36
   <211> 21
   <212> RNA
   <213> Artificial Sequence
<220>
   <223> synthetic FGF9 sense strand small interfering RNA (siRNA)
<400> 36
   uggaugagga cuugccgauu u 21
<210> 37
   <211> 21
   <212> RNA
   <213> Artificial Sequence
<220>
   <223> synthetic FGF9 antisense strand small interfering RNA (siRNA)
<400> 37
   aucggcaagu ccucauccau u 21
<210> 38
   <211> 4
   <212> PRT
   <213> Artificial Sequence
<220>
   <223> synthetic DEAD (Asp-Glu-Ala-Asp) box
<400> 38
   Asp Glu Ala Asp
    1

## Description

### BACKGROUND OF THE INVENTION

Clinical depression, including both bipolar disorders and major depression disorders, is a major public health problem, affecting an estimated 9.5% of the adult population of the United States each year. While it has been hypothesized that mental illness, including mood disorders such as major depression ("MDD") and bipolar disorder ("BP") as well as psychotic disorders such as schizophrenia, may have genetic roots, little progress has been made in identifying gene sequences and gene products that play a role in causing these disorders, as is true for many diseases with a complex genetic origin *(see, e.g.,* Burmeister, Biol. Psychiatry 45:522-532 (1999)).

Anxiety is a normal reaction to stress. It helps one deal with a tense situations, prepare and take exams, or stay focused on an important speech. In general, it helps one cope. When anxiety becomes an excessive, irrational dread of everyday situations, however, it can be disabling. Examples of anxiety disorders include: generalized anxiety disorder, obsessive-compulsive disorder, panic disorder, post-traumatic stress disorder and social phobia (or social anxiety disorder). Anxiety may also be associated with mental disorders such as those described above, including major depression disorder.

Anxiety disorders are considered the most prevalent of psychiatric disorders, 22% of the total population across the seven major pharmaceutical markets suffering from an anxiety disorder, causing considerable individual burden and imposing substantial social burdens, costing the economy over $42 billion in the US alone. However, the exact prevalence of such disorders is difficult to quantify due to the lack of epidemiological data, plus the high incidence of comorbidity with other psychiatric disorders. Furthermore, physicians estimate that only 26% of the population who suffer from an anxiety disorder are diagnosed and therefore treated. This is a result of combination of poor knowledge of the disorder by both patients and physicians.

With approximately 30% of patients requiring second-line therapy, current international guidelines, such as those published by the World Federation of Societies of Biological Psychiatry, recommend the use of selective serotonin re-uptake inhibitors ("SSRIs") as first-line therapy for the treatment of anxiety. Nonetheless, despite the widespread use of SSRIs, approximately 30% of patients require second-line therapy. This suggests that current therapies are ineffective in up to a third of anxiety disorder patients therefore there is a significant need for anxiety drugs with improved efficacy.

### BRIEF SUMMARY OF THE INVENTION

The invention provides FGF9 for use in a method for alleviating one or more symptoms of anxiety in a mammalian subject, said method comprising acutely administering an effective amount of said FGF9 to the subject. In a related embodiment, the effective amount of FGF9 may be administered to the subject by injection. In another related embodiment, the subject is diagnosed with an anxiety disorder before treatment. In yet another related embodiment, the subject has been previously diagnosed with major depression disorder or a similar disorder with anxiety-related symptoms. The effective amount of FGF9 is acutely administered to the subject, for example, when the symptoms of anxiety are deemed severe enough to warrant administration. In a further embodiment, the subject is a human.

In yet another embodiment, the invention provides FGF9 for use in a method for treating an addictive behavior in a mammalian subject, said method comprising chronically administering an effective amount of said FGF9 to said subject. In a related embodiment, the effective amount of FGF9 may be administered to the subject by injection. In another related embodiment, the subject has high FGF2 levels. In a further embodiment, the subject is a human.

As described herein, the present invention relies in part on the surprising discovery that (1) acute administration of FGF2 decreases depression-like behavior and produces an anxiogenic effect, whereas chronic FGF2 administration decreases depression-like behavior and produces an anxiolytic effect; and (2) acute administration of FGF9 produces an anxiolytic effect and increases depression-like behavior, whereas chronic FGF9 administration produces an anxiogenic effect and increases depression-like behavior. Without being bound to any particular theory, these findings indicate that FGF9 may modulate and/or compete with the effects of FGF2 and that FGF9 may be a physiological antagonist of FGF2. As a non-limiting example, FGF9 may counterbalance the effects of FGF2 in patients with high FGF2 levels and reduce risk-taking behavior.

Other objects, features, and advantages of the present invention will be apparent to one of skill in the art from the following detailed description and figures.

### BRIEF DESCRIPTION OF THE DRAWINGS

FIGURE 1 shows FGFR2 variant differences in Mood Disorders. FGFR2 soluble receptor splice variants may represent a smaller percentage of the total receptors in MDD than in controls.
FIGURE 2 shows the effect of acute injection of FGF2 on mouse depression and anxiety, as measured by mobility tests (top) and the elevated plus maze (EPM) test (bottom), respectively. "Open," "center," and "closed" refer to time spent in the open, center and closed parts of the EPM, respectively.
FIGURE 3 shows the effects of injections of NCAM peptide on mouse depression and anxiety, as measured by the climbing and forced swim test (top) and the elevated plus maze (EPM) test (bottom), respectively. "Open," "center," and "closed" refer to time spent in the open, center and closed parts of the EPM, respectively.
FIGURE 4 shows the effects of injections of a peptide inhibitor on mouse depression and anxiety, as measured by the climbing and forced swim test (top) and the elevated plus maze (EPM) test (bottom), respectively. "Open," "center," and "closed" refer to time spent in the open, center and closed parts of the EPM, respectively.
FIGURE 5 shows a table listing genes in the cAMP signaling pathway whose expression is significantly dysregulated in the anterior cingulate cortex (AnCg) from patients with bipolar disorder (BPD).
FIGURE 6 shows a table listing genes in cAMP signaling pathways whose expression is significantly dysregulated in the anterior cingulate cortex (AnCg) of patients with major depression disorder (MDD).
FIGURE 7 shows a table listing genes in the phosphatidylinositol signaling pathway whose expression is significantly dysregulated in the anterior cingulate cortex (AnCg) of patients with bipolar disorder (BPD).
FIGURE 8 shows a table listing genes in the phosphatidylinositol signaling pathway whose expression is significantly dysregulated in the anterior cingulate cortex (AnCg) of patients with major depression disorder (MDD).
FIGURE 9 shows two charts which illustrate the effect of chronic FGF-2 administration (5ng/g, 3 weeks) on anxiety in rodents with different, as measured by the time the rodents spend in the light compartment of the test system. LR, animals with intrinsic high anxiety; HR, animals with intrinsic low anxiety; HRFGF-2, low anxiety animals administered FGF-2; LRFGF-2, high anxiety animals administered FGF-2.
FIGURE 10 shows the inverse relationship between FGF-2 gene expression and anxiety behavior using the "open arms" test. CA-2, hippocampus region CA-2.

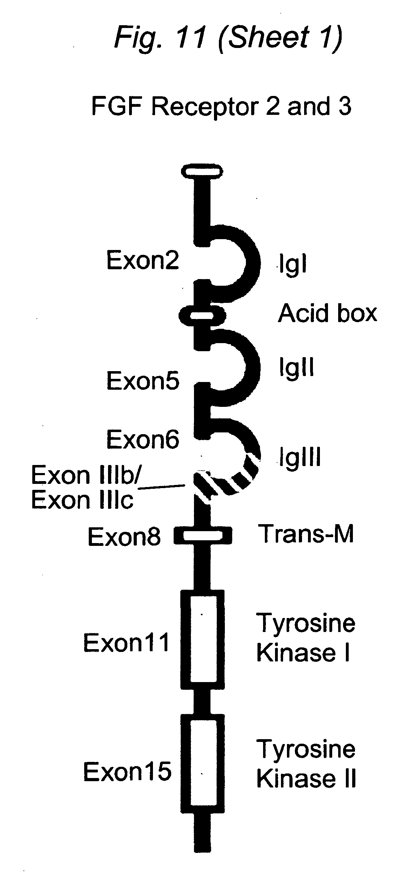

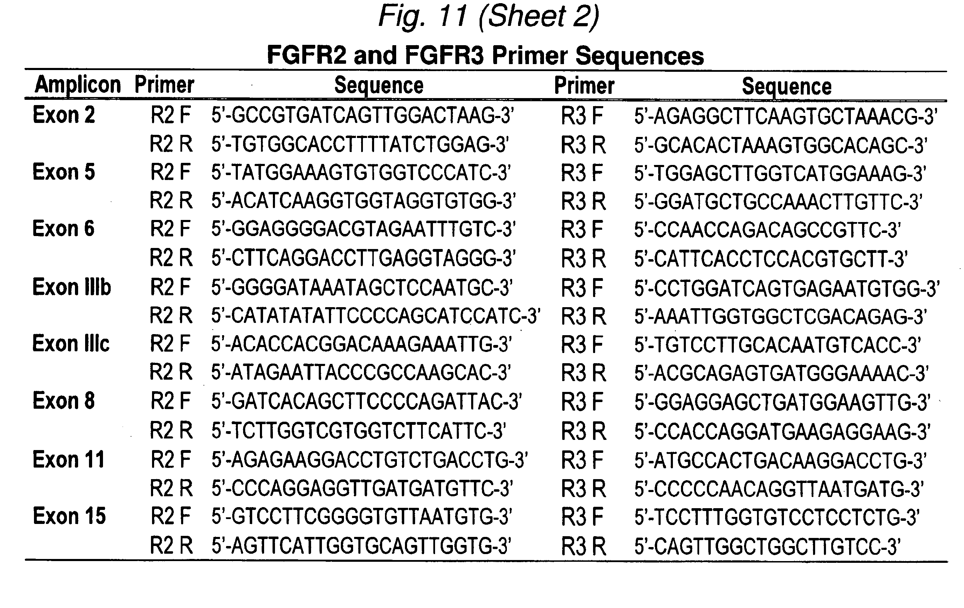

FIGURE 11, top, shows a schematic of the basic structure of FGFR2 and FGFR3 aligned with the exons amplified and described in the Example. Emphasis is placed on the IIIb/IIIc splice variants in the C-terminus of the third Ig-like domain of both receptors (R2 and R3). Exon sequences for FGFR2 and FGFR3 are in no way identical (see FIGURE 11, bottom), but exon nomenclature was synchronized to match each exon number to corresponding regions on both R2 and R3 protein structures. The truncated and cleaved isoforms of the FGF receptors are excluded from the schematic. FIGURE 11, bottom, shows sequences of forward and reverse FGFR2 (SEQ ID NOs: 1-16) and FGFR3 (SEQ ID NOs: 17-32) primers designed for real time RT-PCR quantitative analysis. Primers were optimized and designed for maximum efficiency with differential detection for IIIb/IIIc splice variants for both FGFR2 and FGFR3.
FIGURE 12 shows two charts which illustrate the chronic stress-induced decrease in the exon IIIc:IIIb splice variant expression ratio in both FGFR2 (top panel) and FGFR3 (bottom panel). V (vehicle); NS (non-stress); Chronic stress (S); FGF-2 (F).
FIGURE 13 shows that acute administration of FGF9 (20 ng, i.c.v.) had an anxiolytic effect in rats, as measured by the duration of time spend in the closed arm portion of the test maze versus the open arm portion.
FIGURE 14 shows that chronic administration of FGF9 (20 ng, i.c.v.) daily for 3 weeks increased the depression-like effect seen in the forced swim test compared to acute administration. Chronic FGF9 had an anxiogenic effect on the elevated plus maze task, which is opposite to the anxiolytic effect seen with acute administration.

### DEFINITIONS

A "mental disorder" or "mental illness" or "mental disease" or "psychiatric or neuropsychiatric disease or illness or disorder" refers to mood disorders (*e*.*g*., major depression, mania, and bipolar disorders), psychotic disorders *(e.g.,* schizophrenia, schizoaffective disorder, schizophreniform disorder, delusional disorder, brief psychotic disorder, and shared psychotic disorder), personality disorders, anxiety disorders *(e.g.,* obsessive-compulsive disorder) as well as other mental disorders such as substance -related disorders, childhood disorders, dementia, autistic disorder, adjustment disorder, delirium, multi-infarct dementia, and Tourette's disorder as described in Diagnostic and Statistical Manual of Mental Disorders, Fourth Edition, (DSM IV). Typically, such disorders have a complex genetic and/or a biochemical component.

A "mood disorder" refers to disruption of feeling tone or emotional state experienced by an individual for an extensive period of time. Mood disorders include major depression disorder (i.e., unipolar disorder), mania, dysphoria, bipolar disorder, dysthymia, cyclothymia and many others. *See, e.g.,* Diagnostic and Statistical Manual of Mental Disorders, Fourth Edition, (DSM IV).

"Major depression disorder," "major depressive disorder," or "unipolar disorder" refers to a mood disorder involving any of the following symptoms: persistent sad, anxious, or "empty" mood; feelings of hopelessness or pessimism; feelings of guilt, worthlessness, or helplessness; loss of interest or pleasure in hobbies and activities that were once enjoyed, including sex; decreased energy, fatigue, being "slowed down"; difficulty concentrating, remembering, or making decisions; insomnia, early-morning awakening, or oversleeping; appetite and/or weight loss or overeating and weight gain; thoughts of death or suicide or suicide attempts; restlessness or irritability; or persistent physical symptoms that do not respond to treatment, such as headaches, digestive disorders, and chronic pain. Various subtypes of depression are described in, *e.g.,* DSM IV.

"Bipolar disorder" is a mood disorder characterized by alternating periods of extreme moods. A person with bipolar disorder experiences cycling of moods that usually swing from being overly elated or irritable (mania) to sad and hopeless (depression) and then back again, with periods of normal mood in between. Diagnosis of bipolar disorder is described in, *e.g.,* DSM IV. Bipolar disorders include bipolar disorder I (mania with or without major depression) and bipolar disorder II (hypomania with major depression), see, e.g., DSM IV.

"A psychotic disorder" refers to a condition that affects the mind, resulting in at least some loss of contact with reality. Symptoms of a psychotic disorder include, *e.g.,* hallucinations, changed behavior that is not based on reality, delusions and the like. *See, e.g.,* DSM IV. Schizophrenia, schizoaffective disorder, schizophreniform disorder, delusional disorder, brief psychotic disorder, substance-induced psychotic disorder, and shared psychotic disorder are examples of psychotic disorders.

"Schizophrenia" refers to a psychotic disorder involving a withdrawal from reality by an individual. Symptoms comprise for at least a part of a month two or more of the following symptoms: delusions (only one symptom is required if a delusion is bizarre, such as being abducted in a space ship from the sun); hallucinations (only one symptom is required if hallucinations are of at least two voices talking to one another or of a voice that keeps up a running commentary on the patient's thoughts or actions); disorganized speech *(e.g.,* frequent derailment or incoherence); grossly disorganized or catatonic behavior; or negative symptoms, *i.e.,* affective flattening, alogia, or avolition. Schizophrenia encompasses disorders such as, *e.g.,* schizoaffective disorders. Diagnosis of schizophrenia is described in, *e.g.,* DSM IV. Types of schizophrenia include, *e.g.,* paranoid, disorganized, catatonic, undifferentiated, and residual.

An "antidepressant" refers to an agents typically used to treat clinical depression. Antidepressants includes compounds of different classes including, for example, specific serotonin reuptake inhibitors (e.g., fluoxetine), tricyclic antidepressants (e.g., desipramine), and dopamine reuptake inhibitors (e.g, bupropion). Typically, antidepressants of different classes exert their therapeutic effects via different biochemical pathways. Often these biochemical pathways overlap or intersect. Additional diseases or disorders often treated with antidepressants include, chronic pain, anxiety disorders, and hot flashes.

An "agonist" refers to an agent that binds to a polypeptide or polynucleotide, stimulates, increases, activates, facilitates, enhances activation, sensitizes or up regulates the activity or expression of a polypeptide or polynucleotide.

An "antagonist" refers to an agent that inhibits expression of a polypeptide or polynucleotide or binds to, partially or totally blocks stimulation, decreases, prevents, delays activation, inactivates, desensitizes, or down regulates the activity of a polypeptide or polynucleotide.

"Inhibitors," "activators," and "modulators" of expression or of activity are used to refer to inhibitory, activating, or modulating molecules, respectively, identified using *in vitro and in vivo* assays for expression or activity, *e.g.,* ligands, agonists, antagonists, and their homologs and mimetics. The term "modulator" includes inhibitors and activators. Inhibitors are agents that, *e.g.,* inhibit expression of a polypeptide or polynucleotide or bind to, partially or totally block stimulation or enzymatic activity, decrease, prevent, delay activation, inactivate, desensitize, or down regulate the activity of a polypeptide or polynucleotide, *e.g.,* antagonists. Activators are agents that, *e.g.,* induce or activate the expression of a polypeptide or polynucleotide or bind to, stimulate, increase, open, activate, facilitate, enhance activation or enzymatic activity, sensitize or up regulate the activity of a polypeptide or polynucleotide, *e.g.,* agonists. Modulators include naturally occurring and synthetic ligands, antagonists, agonists, small chemical molecules and the like. Assays to identify inhibitors and activators include, *e.g.,* applying putative modulator compounds to cells, in the presence or absence of a polypeptide or polynucleotide and then determining the functional effects on a polypeptide or polynucleotide activity. Samples or assays comprising a polypeptide or polynucleotide that are treated with a potential activator, inhibitor, or modulator are compared to control samples without the inhibitor, activator, or modulator to examine the extent of effect. Control samples (untreated with modulators) are assigned a relative activity value of 100%. Inhibition is achieved when the activity value of a polypeptide or polynucleotide relative to the control is about 80%, optionally 50% or 25-1 %. Activation is achieved when the activity value of a polypeptide or polynucleotide relative to the control is 110%, optionally 150%, optionally 200-500%, or 1000-3000% higher.

The term "Table #" when used herein includes all sub-tables of the Table referred to *(e.g.,* "Table 1" refers to Table 1A, 1B, and Table 1C) unless otherwise indicated.

"Biological sample" includes sections of tissues such as biopsy and autopsy samples, and frozen sections taken for histologic purposes. Such samples include blood, sputum, tissue, lysed cells, brain biopsy, cultured cells, *e.g.,* primary cultures, explants, and transformed cells, stool, urine, *etc.* A biological sample is typically obtained from a eukaryotic organism, most preferably a mammal such as a primate, *e.g.,* chimpanzee or human; cow; dog; cat; a rodent, *e.g.,* guinea pig, rat, mouse; rabbit; or a bird; reptile; or fish.

The term "gene" means the segment of DNA involved in producing a polypeptide chain; it includes regions preceding and following the coding region (leader and trailer) as well as intervening sequences (introns) between individual coding segments (exons).

The term "isolated," when applied to a nucleic acid or protein, denotes that the nucleic acid or protein is essentially free of other cellular components with which it is associated in the natural state. It is preferably in a homogeneous state although it can be in either a dry or aqueous solution. Purity and homogeneity are typically determined using analytical chemistry techniques such as polyacrylamide gel electrophoresis or high performance liquid chromatography. A protein that is the predominant species present in a preparation is substantially purified. In particular, an isolated gene is separated from open reading frames that flank the gene and encode a protein other than the gene of interest. The term "purified" denotes that a nucleic acid or protein gives rise to essentially one band in an electrophoretic gel. Particularly, it means that the nucleic acid or protein is at least 85% pure, more preferably at least 95% pure, and most preferably at least 99% pure.

The term "nucleic acid" or "polynucleotide" refers to deoxyribonucleotides or ribonucleotides and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues *(*Batzer et al., Nucleic Acid Res. 19:5081 (1991); Ohtsuka et al., J. Biol. Chem. 260:2605-2608 (1985); and Cassol *et al.* (1992); Rossolini et al., Mol. Cell. Probes 8:91-98 (1994)). The term nucleic acid is used interchangeably with gene, cDNA, and mRNA encoded by a gene.

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins *(i.e.,* antigens), wherein the amino acid residues are linked by covalent peptide bonds.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, *e.g.,* hydroxyproline, γ-carboxyglutamate, and O-phosphoserine. Amino acid analogs refers to compounds that have the same basic chemical structure as a naturally occurring amino acid, *i.e.,* an α carbon that is bound to a hydrogen, a carboxyl group, an amino group, and an R group, *e.g.,* homoserine, norleucine, methionine sulfoxide, methionine methyl sulfonium. Such analogs have modified R groups *(e.g.,* norleucine) or modified peptide backbones, but retain the same basic chemical structure as a naturally occurring amino acid. "Amino acid mimetics" refers to chemical compounds that have a structure that is different from the general chemical structure of an amino acid, but that functions in a manner similar to a naturally occurring amino acid.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

The phrase "a nucleic acid sequence encoding" refers to a nucleic acid that contains sequence information for a structural RNA such as rRNA, a tRNA, or the primary amino acid sequence of a specific protein or peptide, or a binding site for a trans-acting regulatory agent. This phrase specifically encompasses degenerate codons *(i.e.,* different codons which encode a single amino acid) of the native sequence or sequences which may be introduced to conform with codon preference in a specific host cell.

The term "recombinant" when used with reference, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under-expressed or not expressed at all.

The term "heterologous" when used with reference to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, *e.g.,* a promoter from one source and a coding region from another source. Similarly, a heterologous protein indicates that the protein comprises two or more subsequences that are not found in the same relationship to each other in nature *(e.g.,* a fusion protein).

An "expression vector" is a nucleic acid construct, generated recombinantly or synthetically, with a series of specified nucleic acid elements that permit transcription of a particular nucleic acid in a host cell. The expression vector can be part of a plasmid, virus, or nucleic acid fragment. Typically, the expression vector includes a nucleic acid to be transcribed operably linked to a promoter.

One who is "predisposed for a mental disorder" as used herein means a person who has an inclination or a higher likelihood of developing a mental disorder when compared to an average person in the general population.

"Anxiety" refers to a physiological and psychological state characterized by any of the following symptoms: heart palpitations, nausea, chest pain, shortness of breath, stomach aches, headaches, panic attacks, sweating, increase in blood pressure, increase in heart rate, increase in the flow of blood to major muscle groups, inhibition of immune and digestive system functions, pale skin, trembling, pupillary dilation, experiencing a sense of dread or panic. An "anxiogenic" substance is one that causes *(e.g.,* brings about, produces, creates, induces) anxiety or increases the level of anxiety in a subject. Anxiogenic effects can be measured by, *e.g.,* the hole-board test in rats and mice *(see, e.g.,* Takeda et al., Eur. J. Pharm., 350:21-29 (1998)). An "anxiolytic" substance is one that may be used for the treatment of symptoms of anxiety in a subject.

"Risk-taking behavior" refers to the voluntary participation in behaviors that contain, or are at least seen to contain, a significant degree of risk. Generally, a risk is considered to be significant if the behavior involves a high degree of risk in comparison with other equivalent behaviors and involves a high degree of actual risk as measured by the probability of death, injury, financial loss, and the like. Examples of risk-taking behavior include, but are not limited to, health risks such as drinking alcohol, taking drugs, driving fast, or engaging in other addictive behaviors; financial risks such as gambling; participation in high risk sports such as mountain climbing or parachuting; and combinations thereof.

The term "administering" includes oral administration, administration as a suppository, topical contact, intravenous, intraperitoneal, intramuscular, intralesional, intrathecal, intranasal or subcutaneous administration, or the implantation of a slow-release device, *e.g.,* a mini-osmotic pump, to a subject. Administration is by any route, including parenteral and transmucosal *(e.g.,* buccal, sublingual, palatal, gingival, nasal, vaginal, rectal, or transdermal). Parenteral administration includes, *e.g.,* intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.* One skilled in the art will know of additional methods for administering an effective amount of a therapeutic of the present invention.

The term "mammalian subject" refers to any mammalian species such as a human, mouse, rat, dog, cat, hamster, guinea pig, rabbit, livestock, and the like.

### DETAILED DESCRIPTION OF THE INVENTION

Evidence based on analysis of only a restricted number of mo lecules suggests altered and unique gene dysregulation that may be involved in the pathophysiology of bipolar disorder (BPD) and major depressive disorder (MDD) as well as in the mechanism of drug treatment for these disorders. The recent development of microarray technology allows a comprehensive view of the mRNA expression profiles of specific genes, systems and signaling pathways.

### I. Introduction

To understand the genetic basis of mental disorders, studies have been conducted to investigate the expression patterns of genes that are differentially expressed specifically in central nervous system of subjects with mood disorders. In several studies, the differential and unique expression of known and novel genes was determined by way of interrogating total RNA samples purified from postmortem brains of BP and MDD patients with Affymetrix Gene Chips® (containing high-density oligonucleotide probe set arrays). The fundamental principle is that by identifying genes and pathways that are differentially expressed in BP and/or MDD (relative to healthy control subjects), via global expression profiling of the transcriptomes as above, one can identify genes that cause, effect, or are associated with the disease, or that interact with drugs used to treat the disease, for use in diagnostic and therapeutic applications.

The Examples provided herein describe the microarray gene expression profiling of the dorsolateral prefrontal, anterior cingulate, hippocampus, Nucleus Accumbens, Amigdala and cerebellar cortices of BPD and MDD patients. In particular, the mRNA expression levels of genes related to the FGF and GPCRs pathways are disclosed. The detection of splice variants of FGFR2 is also disclosed, which are also dysregulated. Genes which are dysregulated by lithium for the specific treatment of BP disorders are also provided.

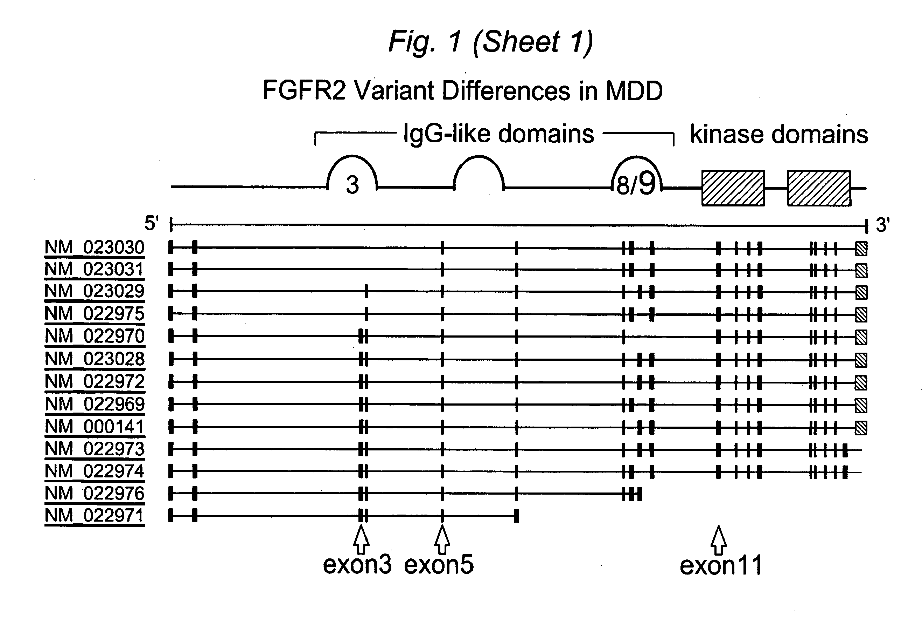

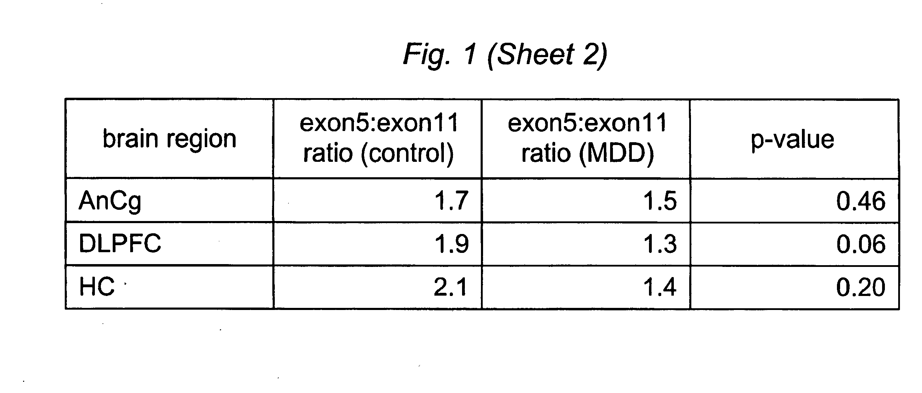

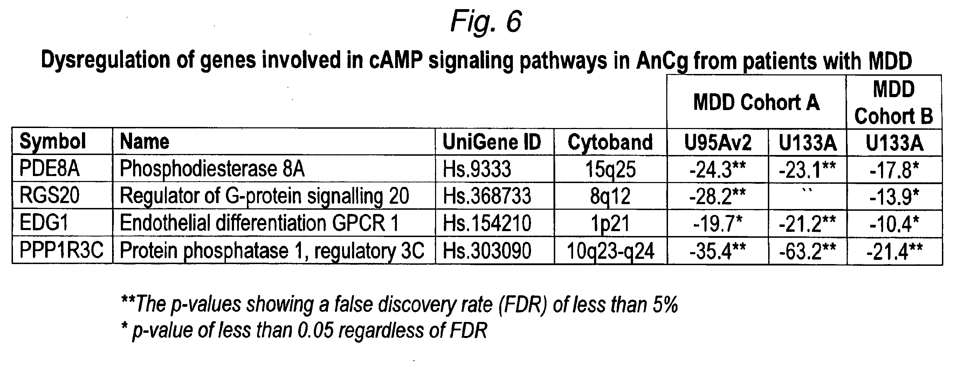

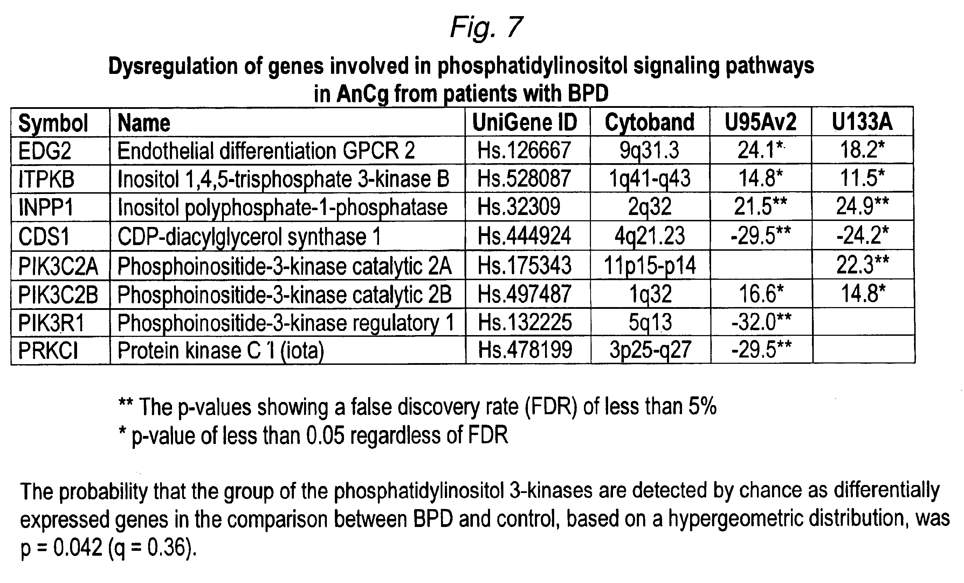

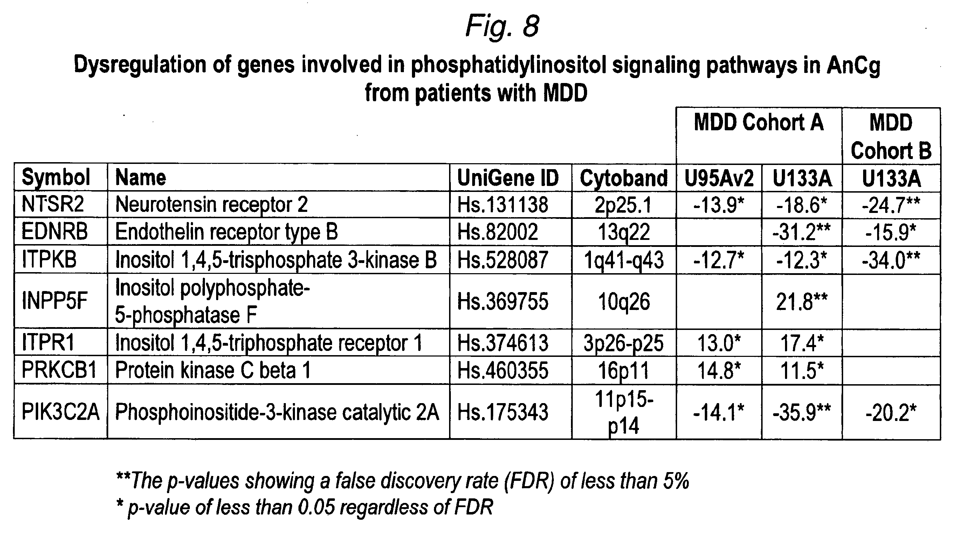

Methods for exploiting the altered expression (either higher or lower expression as indicated herein) or unique differential expression of the genes of FIGURE 1, FIGURES 5-8, or Tables 1-4 which is observed in selected brain regions of patients diagnosed with mood disorders *(e.g.,* bipolar disorder and major depression disorder) in comparison with normal individuals are provided.

The invention provides FGF9 for use in methods of treating patients with anxiety or addictive behaviour, *e.g.,* by administering the FGF9 of the invention.

Antidepressants belong to different classes, *e.g.,* desipramine, bupropion, and fluoxetine are in general equally effective for the treatment of clinical depression, but act by different mechanisms. The similar effectiveness of the drugs for treatment of mood disorders suggests that they act through a presently unidentified common pathway. Animal models of depression, including treatment of animals with known therapeutics such as SSRIs, can be used to examine the mode of action of the genes provided herein. Lithium is drug of choice for treating BP.

### II. General Recombinant Nucleic Acid Methods

Polynucleotides may be isolated and cloned using recombinant methods. Such polynucleotides include, *e.g.,* those listed in FIGURE 1, FIGURES 5-8, or Tables 1-4, which can be used for, *e.g.,* protein expression or during the generation of variants, derivatives, expression cassettes, to monitor gene expression, for the isolation or detection of sequences in different species, for diagnostic purposes in a patient, *e.g.,* to detect mutations or to detect expression levels of nucleic acids or polypeptides. The sequences may be operably linked to a heterologous promoter. The nucleic acids may be from any mammal, including, in particular, *e.g.,* a human, a mouse, a rat, a primate, *etc.*

### A. General Recombinant Nucleic Acids Methods

Routine techniques in the field of recombinant genetics are relied on. Basic texts disclosing the general methods include Sambrook et al., Molecular Cloning, A Laboratory Manual (3rd ed. 2001); Kriegler, Gene Transfer and Expression: A Laboratory Manual (1990); and Current Protocols in Molecular Biology (Ausubel et al., eds., 1994)).

For nucleic acids, sizes are given in either kilobases (kb) or base pairs (bp). These are estimates derived from agarose or acrylamide gel electrophoresis, from sequenced nucleic acids, or from published DNA sequences. For proteins, sizes are given in kilodaltons (kDa) or amino acid residue numbers. Proteins sizes are estimated from gel electrophoresis, from sequenced proteins, from derived amino acid sequences, or from published protein sequences.

Oligonucleotides that are not commercially available can be chemically synthesized according to the solid phase phosphoramidite triester method first described by Beaucage & Caruthers, Tetrahedron Letts. 22:1859-1862 (1981), using an automated synthesizer, as described in Van Devanter et. al., Nucleic Acids Res. 12:6159-6168 (1984). Purification of oligonucleotides is by either native acrylamide gel electrophoresis or by anion-exchange HPLC as described in Pearson & Reanier, J. Chrom. 255:137-149 (1983).

The sequence of the cloned genes and synthetic oligonucleotides can be verified after cloning using, *e.g.,* the chain termination method for sequencing double-stranded templates of Wallace et al., Gene 16:21-26 (1981).

### B. Cloning Methods for the Isolation of Nucleotide Sequences Encoding Desired Proteins

In general, the nucleic acids encoding the subject proteins are cloned from DNA sequence libraries that are made to encode cDNA or genomic DNA. The particular sequences can be located by hybridizing with an oligonucleotide probe, the sequence of which can be derived from the sequences of the genes listed in FIGURE 1, FIGURES 5-8, or Tables 1-4, which provide a reference for PCR primers and defines suitable regions for isolating specific probes. Alternatively, where the sequence is cloned into an expression library, the expressed recombinant protein can be detected immunologically with antisera or purified antibodies made against a polypeptide comprising an amino acid sequence encoded by a gene listed in FIGURE 1, FIGURES 5-8, or Tables 1-4.

Methods for making and screening genomic and cDNA libraries are well known to those of skill in the art *(see, e.g.,* Gubler and Hoffman Gene 25:263-269 (1983); Benton and Davis Science, 196:180-182 (1977); and Sambrook, *supra*). Brain cells are an example of suitable cells to isolate RNA and cDNA sequences.

Briefly, to make the cDNA library, one should choose a source that is rich in mRNA. The mRNA can then be made into cDNA, ligated into a recombinant vector, and transfected into a recombinant host for propagation, screening and cloning. For a genomic library, the DNA is extracted from a suitable tissue and either mechanically sheared or enzymatically digested to yield fragments of preferably about 5-100 kb. The fragments are then separated by gradient centrifugation from undesired sizes and are constructed in bacteriophage lambda vectors. These vectors and phage are *packaged in vitro,* and the recombinant phages are analyzed by plaque hybridization. Colony hybridization is carried out as generally described in Grunstein et al., Proc. Natl. Acad. Sci. USA., 72:3961-3965 (1975).

An alternative method combines the use of synthetic oligonucleotide primers with polymerase extension on an mRNA or DNA template. Suitable primers can be designed from specific sequences. This polymerase chain reaction (PCR) method amplifies the nucleic acids encoding the protein of interest directly from mRNA, cDNA, genomic libraries or cDNA libraries. Restriction endonuclease sites can be incorporated into the primers. Polymerase chain reaction or other *in vitro* amplification methods may also be useful, for example, to clone nucleic acids encoding specific proteins and express said proteins, to synthesize nucleic acids that will be used as probes for detecting the presence of mRNA encoding a polypeptide in physiological samples, for nucleic acid sequencing, or for other purposes *(see,* U.S. Patent Nos. 4,683,195 and 4,683,202). Genes amplified by a PCR reaction can be purified from agarose gels and cloned into an appropriate vector.

Appropriate primers and probes for identifying polynucleotides from mammalian tissues can be derived from the sequences provided herein. For a general overview of PCR, *see,* Innis et al. PCR Protocols: A Guide to Methods and Applications, Academic Press, San Diego (1990).

Synthetic oligonucleotides can be used to construct genes. This is done using a series of overlapping oligonucleotides, usually 40-120 bp in length, representing both the sense and anti-sense strands of the gene. These DNA fragments are then annealed, ligated and cloned.

A gene encoding a polypeptide can be cloned using intermediate vectors before transformation into mammalian cells for expression. These intermediate vectors are typically prokaryote vectors or shuttle vectors. The proteins can be expressed in either prokaryotes, using standard methods well known to those of skill in the art, or eukaryotes as described *infra.*

### III. Purification of Proteins

Either naturally occurring or recombinant polypeptides can be purified for use in functional assays. Naturally occurring polypeptides, e.g., FGF9, and/or polypeptides encoded by genes listed in FIGURE 1, FIGURES 5-8, or Tables 1-4, can be purified, for example, from mouse or human tissue such as brain or any other source of an ortholog. Recombinant polypeptides can be purified from any suitable expression system.

The polypeptides may be purified to substantial purity by standard techniques, including selective precipitation with such substances as ammonium sulfate; column chromatography, immunopurification methods, and others *(see, e.g.,* Scopes, Protein Purification: Principles and Practice (1982); U.S. Patent No. 4,673,641; Ausubel *et al., supra;* and Sambrook *et al., supra*).

A number of procedures can be employed when recombinant polypeptides are purified. For example, proteins having established molecular adhesion properties can be reversible fused to polypeptides. With the appropriate ligand, the polypeptides can be selectively adsorbed to a purification column and then freed from the column in a relatively pure form. The fused protein is then removed by enzymatic activity. Finally the polypeptide can be purified using immunoaffinity columns.

### A. Purification of Proteins from Recombinant Bacteria

When recombinant proteins are expressed by the transformed bacteria in large amounts, typically after promoter induction, although expression can be constitutive, the proteins may form insoluble aggregates. There are several protocols that are suitable for purification of protein inclusion bodies. For example, purification of aggregate proteins (hereinafter referred to as inclusion bodies) typically involves the extraction, separation and/or purification of inclusion bodies by disruption of bacterial cells typically, but not limited to, by incubation in a buffer of about 100-150 µg/ml lysozyme and 0.1% Nonidet P40, a non-ionic detergent. The cell suspension can be ground using a Polytron grinder (Brinkman Instruments, Westbury, NY). Alternatively, the cells can be sonicated on ice. Alternate methods of lysing bacteria are described in Ausubel *et al.* and *Sambrook et al.,* both *supra,* and will be apparent to those of skill in the art.

The cell suspension is generally centrifuged and the pellet containing the inclusion bodies resuspended in buffer which does not dissolve but washes the inclusion bodies, e.g., 20 mM Tris-HCl (pH 7.2), 1 mM EDTA, 150 mM NaCl and 2% Triton-X 100, a non-ionic detergent. It may be necessary to repeat the wash step to remove as much cellular debris as possible. The remaining pellet of inclusion bodies may be resuspended in an appropriate buffer (e.g., 20 mM sodium phosphate, pH 6.8, 150 mM NaCl). Other appropriate buffers will be apparent to those of skill in the art.

Following the washing step, the inclusion bodies are solubilized by the addition of a solvent that is both a strong hydrogen acceptor and a strong hydrogen donor (or a combination of solvents each having one of these properties). The proteins that formed the inclusion bodies may then be renatured by dilution or dialysis with a compatible buffer. Suitable solvents include, but are not limited to, urea (from about 4 M to about 8 M), formamide (at least about 80%, volume/volume basis), and guanidine hydrochloride (from about 4 M to about 8 M). Some solvents that are capable of solubilizing aggregate-forming proteins, such as SDS (sodium dodecyl sulfate) and 70% formic acid, are inappropriate for use in this procedure due to the possibility of irreversible denaturation of the proteins, accompanied by a lack of immunogenicity and/or activity. Although guanidine hydrochloride and similar agents are denaturants, this denaturation is not irreversible and renaturation may occur upon removal (by dialysis, for example) or dilution of the denaturant, allowing re-formation of the immunologically and/or biologically active protein of interest. After solubilization, the protein can be separated from other bacterial proteins by standard separation techniques.

Alternatively, it is possible to purify proteins from bacteria periplasm. Where the protein is exported into the periplasm of the bacteria, the periplasmic fraction of the bacteria can be isolated by cold osmotic shock in addition to other methods known to those of skill in the art *(see, Ausubel et al., supra*). To isolate recombinant proteins from the periplasm, the bacterial cells are centrifuged to form a pellet. The pellet is resuspended in a buffer containing 20% sucrose. To lyse the cells, the bacteria are centrifuged and the pellet is resuspended in ice-cold 5 mM MgSO₄ and kept in an ice bath for approximately 10 minutes. The cell suspension is centrifuged and the supernatant decanted and saved. The recombinant proteins present in the supernatant can be separated from the host proteins by standard separation techniques well known to those of skill in the art.

### B. Standard Protein Separation Techniques For Purifying Proteins

### 1. Solubility Fractionation

Often as an initial step, and if the protein mixture is complex, an initial salt fractionation can separate many of the unwanted host cell proteins (or proteins derived from the cell culture media) from the recombinant protein of interest. The preferred salt is ammonium sulfate. Ammonium sulfate precipitates proteins by effectively reducing the amount of water in the protein mixture. Proteins then precipitate on the basis of their solubility. The more hydrophobic a protein is, the more likely it is to precipitate at lower ammonium sulfate concentrations. A typical protocol is to add saturated ammonium sulfate to a protein solution so that the resultant ammonium sulfate concentration is between 20-30%. This will precipitate the most hydrophobic proteins. The precipitate is discarded (unless the protein of interest is hydrophobic) and ammonium sulfate is added to the supernatant to a concentration known to precipitate the protein of interest. The precipitate is then solubilized in buffer and the excess salt removed if necessary, through either dialysis or diafiltration. Other methods that rely on solubility of proteins, such as cold ethanol precipitation, are well known to those of skill in the art and can be used to fractionate complex protein mixtures.

### 2. Size Differential Filtration

Based on a calculated molecular weight, a protein of greater and lesser size can be isolated using ultrafiltration through membranes of different pore sizes (for example, Amicon or Millipore membranes). As a first step, the protein mixture is ultrafiltered through a membrane with a pore size that has a lower molecular weight cut-off than the molecular weight of the protein of interest. The retentate of the ultrafiltration is then ultrafiltered against a membrane with a molecular cut off greater than the molecular weight of the protein of interest. The recombinant protein will pass through the membrane into the filtrate. The filtrate can then be chromatographed as described below.

### 3. Column Chromatography

The proteins of interest can also be separated from other proteins on the basis of their size, net surface charge, hydrophobicity and affinity for ligands. In addition, antibodies raised against proteins can be conjugated to column matrices and the proteins immunopurified. All of these methods are well known in the art.

It will be apparent to one of skill that chromatographic techniques can be performed at any scale and using equipment from many different manufacturers *(e.g.,* Pharmacia Biotech).

### IV. Administration and Pharmaceutical Compositions

Modulators of the polynucleotides or polypeptides disclosed herein *(e.g.,* antagonists or agonists, such as FGF9, FGF2, NCAM, peptide inhibitors of the FGF system, or siRNA and/or antisense inhibitors of genes which are overexpressed in subjects with mental disorders) can be administered directly to a mammalian subject for modulation of activity of those *molecules in vivo.* Administration is by any of the routes normally used for introducing a modulator compound into ultimate contact with the tissue to be treated and is well known to those of skill in the art. Although more than one route can be used to administer a particular composition, a particular route can often provide a more immediate and more effective reaction than another route.

Diseases that can be treated include the following, which include the corresponding reference number from Morrison, *DSM-IV Made Easy,* 1995: Schizophrenia, Catatonic, Subchronic, (295.21); Schizophrenia, Catatonic, Chronic (295.22); Schizophrenia, Catatonic, Subchronic with Acute Exacerbation (295.23); Schizophrenia, Catatonic, Chronic with Acute Exacerbation (295.24); Schizophrenia, Catatonic, in Remission (295.55); Schizophrenia, Catatonic, Unspecified (295.20); Schizophrenia, Disorganized, Subchronic (295.11); Schizophrenia, Disorganized, Chronic (295.12); Schizophrenia, Disorganized, Subchronic with Acute Exacerbation (295.13); Schizophrenia, Disorganized, Chronic with Acute Exacerbation (295.14); Schizophrenia, Disorganized, in Remission (295.15); Schizophrenia, Disorganized, Unspecified (295.10); Schizophrenia, Paranoid, Subchronic (295.31); Schizophrenia, Paranoid, Chronic (295.32); Schizophrenia, Paranoid, Subchronic with Acute Exacerbation (295.33); Schizophrenia, Paranoid, Chronic with Acute Exacerbation (295.34); Schizophrenia, Paranoid, in Remission (295.35); Schizophrenia, Paranoid, Unspecified (295.30); Schizophrenia, Undifferentiated, Subchronic (295.91); Schizophrenia, Undifferentiated, Chronic (295.92); Schizophrenia, Undifferentiated, Subchronic with Acute Exacerbation (295.93); Schizophrenia, Undifferentiated, Chronic with Acute Exacerbation (295.94); Schizophrenia, Undifferentiated, in Remission (295.95); Schizophrenia, Undifferentiated, Unspecified (295.90); Schizophrenia, Residual, Subchronic (295.61); Schizophrenia, Residual, Chronic (295.62); Schizophrenia, Residual, Subchronic with Acute Exacerbation (295.63); Schizophrenia, Residual, Chronic with Acute Exacerbation (295.94); Schizophrenia, Residual, in Remission (295.65); Schizophrenia, Residual, Unspecified (295.60); Delusional (Paranoid) Disorder (297.10); Brief Reactive Psychosis (298.80); Schizophreniform Disorder (295.40); Schizoaffective Disorder (295.70); Induced Psychotic Disorder (297.30); Psychotic Disorder NOS (Atypical Psychosis) (298.90); Personality Disorders, Paranoid (301.00); Personality Disorders, Schizoid (301.20); Personality Disorders, Schizotypal (301.22); Personality Disorders, Antisocial (301.70); Personality Disorders, Borderline (301.83) and bipolar disorders, maniac, hypomaniac, dysthymic or cyclothymic disorders, substance-induced mood disorders, major depression, psychosis, including paranoid psychosis, catatonic psychosis, delusional psychosis, having schizoaffective disorder, various anxiety disorders, and substance-induced psychotic disorder.

Modulators of polynucleotides or polypeptides may be combined with other drugs useful for treating mental disorders including useful for treating mood disorders, e.g., schizophrenia, bipolar disorders, major depression, and/or anxiety disorders. Pharmaceutical compositions may comprise a modulator of a polypeptide of polynucleotide combined with at least one of the compounds useful for treating schizophrenia, bipolar disorder, or major depression, *e.g.,* such as those described in U.S. Patent Nos. 6,297,262; 6,284,760; 6,284,771; 6,232,326; 6,187,752; 6,117,890; 6,239,162 or 6,166,008. In the invention, FGF9 is used to treat anxiety disorders and symptoms relating to anxiety that may be associated with the above-mentioned disorders *(e.g.,* major depression disorder).

The pharmaceutical compositions of the invention may comprise a pharmaceutically acceptable carrier. Pharmaceutically acceptable carriers are determined in part by the particular composition being administered, as well as by the particular method used to administer the composition. Accordingly, there is a wide variety of suitable formulations of pharmaceutical compositions of the present invention *(see, e.g.,* Remington's Pharmaceutical Sciences, 17th ed. 1985)).

The modulators *(e.g.,* agonists or antagonists) of the expression or activity of the a polypeptide or polynucleotide, alone or in combination with other suitable components, can be made into aerosol formulations *(i.e.,* they can be "nebulized") to be administered via inhalation or in compositions useful for injection. Aerosol formulations can be placed into pressurized acceptable propellants, such as dichlorodifluoromethane, propane, nitrogen, and the like.

Formulations suitable for administration include aqueous and non-aqueous solutions, isotonic sterile solutions, which can contain antioxidants, buffers, bacteriostats, and solutes that render the formulation isotonic, and aqueous and non-aqueous sterile suspensions that can include suspending agents, solubilizers, thickening agents, stabilizers, and preservatives. In the practice of this invention, compositions can be administered, for example, orally, nasally, topically, intravenously, intraperitoneally, or intrathecally. The formulations of compounds can be presented in unit-dose or multi-dose sealed containers, such as ampoules and vials. Solutions and suspensions can be prepared from sterile powders, granules, and tablets of the kind previously described. The modulators can also be administered as part of a prepared food or drug.

The dose administered to a patient, in the context of the present invention should be sufficient to effect a beneficial response in the subject over time. The optimal dose level for any patient will depend on a variety of factors including the efficacy of the specific modulator employed, the age, body weight, physical activity, and diet of the patient, on a possible combination with other drugs, and on the severity of the disorder. The size of the dose also will be determined by the existence, nature, and extent of any adverse side effects that accompany the administration of a particular compound or vector in a particular subject.

In determining the effective amount of the modulator to be administered a physician may evaluate circulating plasma levels of the modulator, modulator toxicity, and the production of anti-modulator antibodies. In general, the dose equivalent of a modulator is from about 1 ng/kg to 10 mg/kg for a typical subject.

For administration, modulators can be administered at a rate determined by the LD-50 of the modulator, and the side effects of the modulator at various concentrations, as applied to the mass and overall health of the subject. Administration can be accomplished via single or divided doses.

### V. Examples

### Example 1: Differential Expression of Genes Associated with Suicide in Both BP and MDD Subjects

Previous studies have investigated genes associated with mood disorders and suicidal tendencies, using microarrays and PCRs to analzye gene expression (Sibille *et al.,* 2004; Yanagi M, et al., J Hum Genet., 50(4):210-6(2005)). Neither investigation, however, used a stringent analysis of suicide compared to mood disorder and suicide compared to controls to detect genes that might be most representative of suicide. This Example describes microarray gene expression profiles in the amygdala, anterior cingulate, and cerebellum in postmortem brains from BPD and MDD patients that committed suicide, focusing on mRNA expression levels of the molecules which regulate white-matter, oligodendrocyte, myelin, and other pathways. The genes identified here may be used as biomarkers for detecting and treating suicidal behavior.

Genes were discovered by selecting subjects run on U133P chips with mRNA quality > 1.4, pH>6.6, and AFS=0. Suicide victims with a mood disorder (n = 14) were compared to non-suicide victims suffering from a mood disorder (n =9) and controls (n =27). The age and pH were different between groups, and were entered as a covariate in ANCOVA. Myelin and oligodendrocyte gene expression were found to be dysregulated in suicidal mood disorder subjects compared to non-suicidal mood disorder subjects or controls in the amygdala. The complete list of identified genes which were dysregulated in suicidal patients versus non-suicidal mood disorder patients is presented in Table 1A. Table 1B lists genes which were dysregulated in suicidal MDD patients versus non-suicidal MDD patients, and genes which were dysregulated in suicidal MDD patients versus control patients.

A similar study was performed using brains of MDD subjects who were known to be drug abusers and comparing gene expression in those subjects to gene expression in MDD subjects who were not substance abusers, as well as to control subjects. Table 1C is a list of genes which were shown to be dysregulated in substance-abusing MDD patients versus MDD patients who were not substance abusers. Table 1C also shows genes which were dysregulated in substance-abusing MDD patients versus control subjects.

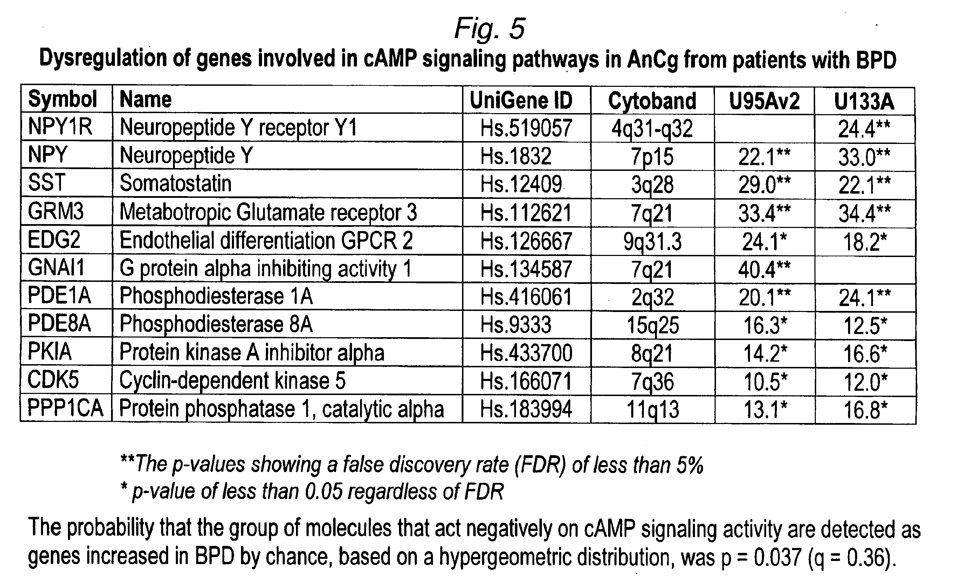

In a related study, two cohorts were used to study and compare gene expression in BP and MDD patients versus normal patients. Cohort A consisted of 7 controls, 6 BPD patients, and 9 MDD patients. Cohort B included 7 controls and 5 MDD patients. The subjects were selected to avoid possible confounding effects of agonal events, tissue pH, RNA integrity, gender and age. The results, summarized in Figures 5-8, show that changes were observed in the expression levels of GPCRs and molecules regulating cAMP- and phosphatidylinositol signaling pathways in the cerebral cortices, especially in the anterior cingulate cortex, of mood disorder patients. Expression levels of molecules acting as negative regulators in cAMP signaling were increased in BPD, while molecules activating cAMP signaling were not altered. Contrasted with the changes in BPD, molecules suppressing cAMP signaling were decreased in MDD. Expression levels of inositol polyphosphate-1-phosphatase and phosphatidylinositol 3-kinases were altered in BPD, while protein kinase C beta-1, inositol triphosphate receptor-1, inositol polyphosphate-5-phosphatase were increased in MDD. Two orphan GPCR genes, GPRC5B and GPR37, consistently showed significant decreases in the three cortices in MDD, and significant increases in anterior cingulate cortex of BPD. Measuring differences in the expression of the genes identifed in Figures 5-8 is a useful tool for determining whether a subject is suffering from a particular mental illness, particularly BP or MDD.

### Example 2: Identification of Lithium Responsive Genes which are Dysregulated in BPD

This Example demonstrates that certain genes in non-human primates (healthy rhesus macaque monkey) are differentially expressed in response to treatment with the mood-stabilizing drug, lithium (Li), the drug of choice for the treatment of BP. Gene expression profiling was carried out on the anterior cingulate cortex (AnCg), dorsolateral prefrontal cortex (DLPFC), hippocampus (HC) and amygdala (AMY) of rhesus macaque monkeys, using the gene expression detection methods described herein, and compared to the human postmortem results described above. Table 2A shows the lithium-responsive genes which had been previously identified in the literature and which were confirmed by the present investigation. Table 2B shows genes that are newly identified as lithium-responsive in primates and which are also dysregulated in human subjects with bipolar disorder.

### Example 3: FGFR2 Variant Differences in Mood Disorders

The FGF receptor 2 (FGFR2) transcript is consistently found to be decreased in several brain areas of depressed subjects *(see, e.g.,* U.S. Pat. App. No. 10/701,263, filed Nov. 3, 2003, published as U.S. Pat. Publ. No. 20040152111-A1 on August 5, 2004). The human FGFR2 gene contains 19 exons and produces as many as 13 splice variants. These variants fall into three main functional classes: first, variants that lack the transmembrane and tyrosine kinase domain which are thought to be soluble receptors; second, variants that contain the Ig IIIc type domain encoded by exon 9; and third, variants that contain the Ig IIIb type domain encoded by exon 8. The Ig III type domain confers ligand specificity and thus these latter two variants have different pharmacological profiles based on their use of the IIIc or IIIb domain. This Example describes PCR-based measurements of exons present in total RNA derived from human cortex (dorsolateral prefrontal and anterior cingulated) and hippocampus.

**Methods** Post-mortem human brans were obtained and dissected as previously described (Evans et al., PNAS 101(43):15506-11 (2004)). RNA for microarray analysis and semi-quantitative RT-PCR was extracted from discrete brain regions using Trizol.

Microarray data was generated with a combination of Affymetrix 133A anti 133plus 2.0 chips and was analyzed using a custom probe mapping file based on a recent generation of the RefSeq database (http://brainarray.mbni.med.umich.edu/Brainarray/Database/CustomCDF). Each biological sample was run independently at two sites (University of California-Irvine, University of run California-Davis or the University of Michigan). Probe set signals were calculated using RMA (Bolstad et al., Bioinformatics 19(2):185-93 (2003)) and statistical comparisons were made after median centering the RMA data separately for each technical block (across independent cohorts and sites). P-values were constructed from t-tests between cases and controls. The final subject composition included 13 major depressive subjects and 16 controls. All were free of agonal factors, had brain pH measurements greater than 6.8, and met other quality measures.

Semi-quantitative RT-PCR data were generated with exon specific primers and the SVBR green method using the BioRad iCycler. All primer pairs used in quantitative reactions were tested for efficiency and determined to be at approximately 100%. Cycle threshold (Ct) values were chosen within the linear range of amplification and were normalized to total cDNA concentration as determined by the PicoGreen assay (Molecular Probes). Contaminating genomic DNA was eliminated with DNAse prior to cDNA synthesis with a mixture of random hexamers and poly-T primers and was confirmed eliminated by amplifying fragments across smaller introns (axon 3 to exon 4 and exon 7 to exon 10). No intron-containing amplicons were detected.

**Results** The results of the above-mentioned study are partially summarized in FIGURE 1, which shows the differential expression of exons 5 and 11 in depressed versus control subjects. More specifically, the results show that the ratio of expression of exon 5 to exon 11 is significantly lowered in MDD patients, particularly in the DLPFC region. A similar analysis of the expression of exon 9 (coding for the IIIc variant) showed that exon 9 expression in the AnCg and HC regions was decreased in MDD subjects.

### Example 4: Effect of Injection of FGF2, FGL Peptide (NCAM), and Peptide Inhibitor of FGF Receptors on the Behavior of Rodents

### A. Microinjection of FGF2

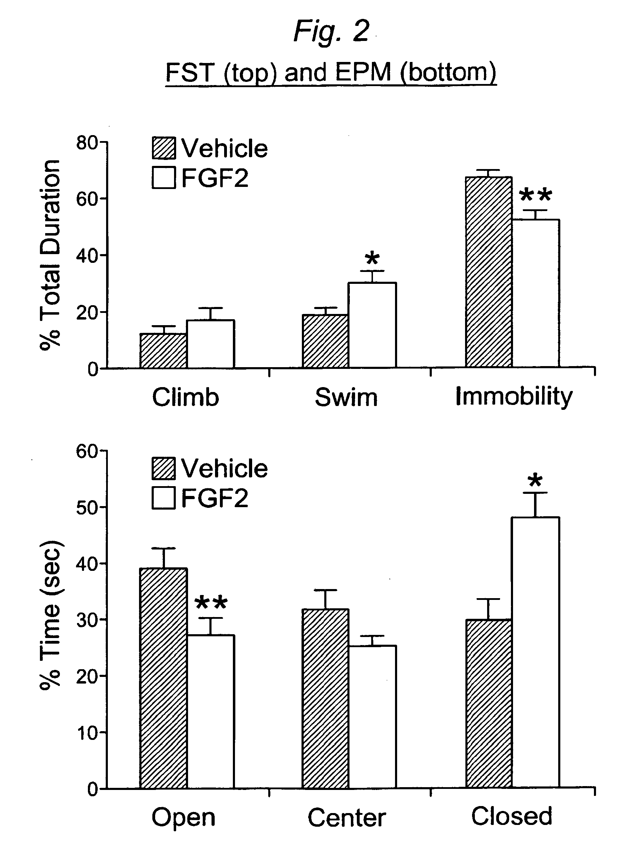

This set of experiments shows significant effects following the microinjection of FGF2, using both the forced swim test (FST) and the elevated plus maze (EPM) to evaluate depression in the subject animals. In the FST, the FGF2-injected (n = 12) animals exhibited more swimming (t[23] = 2.20, *p* < 0.05) and less immobility (t[23] = 2.88, *p* < 0.01) than controls (n = 13). This is indicative of less depression-like behavior in FGF2 animals. However, in the EPM, the FGF2-injected animals spent significantly more time in the closed arms (t[13] = 3.18, *p*<0.01) and less time in the open arms (t[13] = 2.46, *p* < 0.05). These results (FIGURE 2) show that anxiety-like behavior is increased after an acute injection of FGF2.

### B. Microinjection of NCAM (FGL peptide)

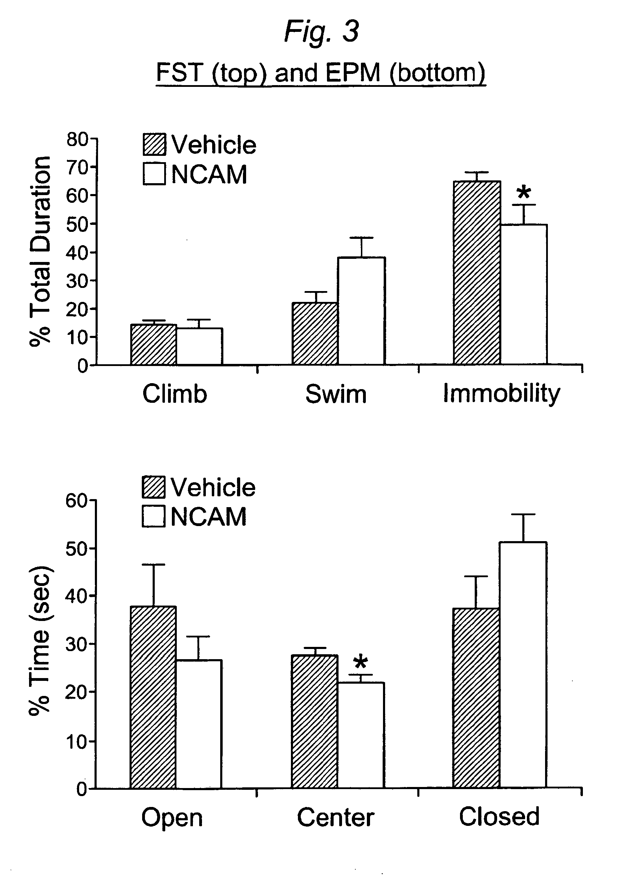

This set of experiments shows a significant effect on animal mood in the forced swim test after NCAM administration (amino acid sequence = EVYVVAENQQGKSKA (SEQ ID NO:34); *see* FIGURE 3). Here, the NCAM-injected animals (n = 13) exhibited less immobility (t[24] = 2.13, *p* < 0.05) than controls (n = 13). Again, this is an index of less depression-like behavior. This is also in the same direction as the FGF2 data, consistent with the fact that both FGF2 and NCAM interact with the FGF receptor. Similarly, the NCAM-injected animals spent more time (although not significantly more time) in the closed arms and less time in the open arms, consistent with increased anxiety-like behavior. NCAM-injected animals also spent significantly less time in the center quadrant (t[18] = 2.40, *p* < 0.05).

### C. Microinjection of a Peptide Inhibitor of FGFR

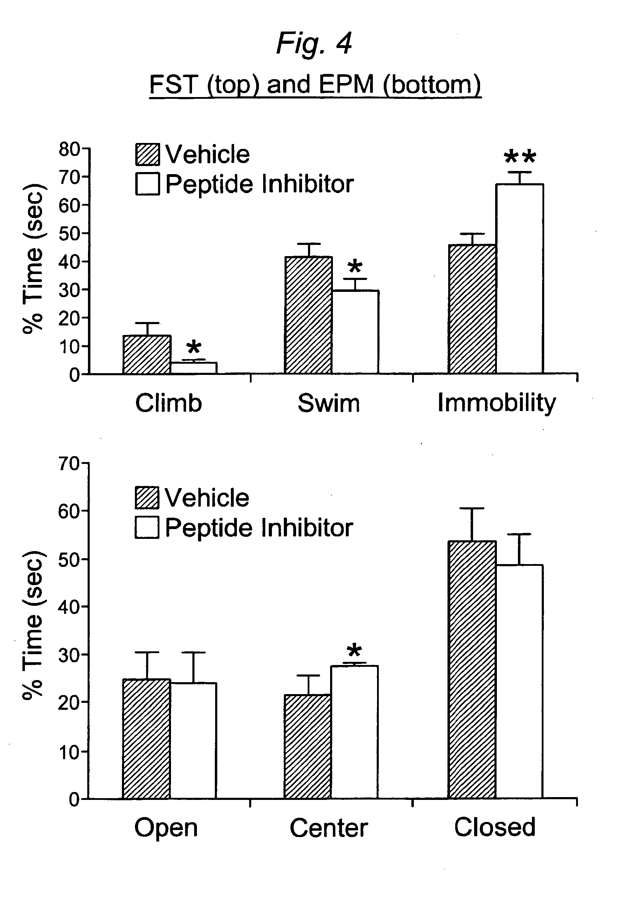

This set of experiments shows a significant effect on animal mood, using both the forced swim test and the elevated plus maze test, after injection with an FGF system peptide inhibitor (amino acid sequence = HFKDPKRLY; SEQ ID NO: 35). The results are shown in FIGURE 4. In the FST, the inhibitor-injected animals (n=7) exhibited significantly less climbing (t[12] = 2.06, *p* < 0.05), less swimming (t[12] = 1.92, *p* <0.05) and more immobility (t[12] = 3.58, *p* < 0.005) than controls (n = 7). These results show that inhibition of the FGF system can result in increased depression-like behavior. These results confirm and advance the results of the previous data sets, and are consistent with studies of the postmortem tissue of individuals with major depression. The inhibitor-injected animals also spent significantly more time in the center quadrant of the EPM (T[7,7] = 35.0, *p* = 0.03). Although the same animals spent less time in the closed arms and equal time in the open arms, indicative of increased anxiety-like behavior, the lengths of time spent were not-significantly different. These observations are nevertheless consistent with the conclusions drawn from the microinjection studies above.

### D. Administration of FGF2 Induces Long-Term Changes in Hippocampal Gene Expression

**Methods.** Sprague- Dawley rats were injected with either vehicle or FGF2 (20ng/g, s.c.) the day after birth and sacrificed after Morris water maze testing as adults. We assessed changes in gene expression using both a candidate approach and a gene discovery approach with laser-capture microdissection of the dentate gyrus followed by microarray analyses.

**Results.** Rats injected with FGF2 performed significantly better in learning and memory tests (e.g., 20 seconds on average to find a hidden platform in Morris Water maze test versus 25 seconds on average for vehicle-injected rats). Several genes associated with neural plasticity were also found altered in the adult rats, as shown by histochemical and RNA expression assays. For example, expression of GAP-43, Rgs4, trkB, CCK, SST, and Vgfwas increased, while expression of NCAM was decreased.

### Example 5: Anxiolytic Effect of Chronically Administered FGF2

Anxiety disorders have a high comorbidity with other neuropsychiatric disorders including Major Depression (MD). This Example shows that chronic FGF2 administration has an anxiolytic effect in,rats. Rats were placed into "high anxiety" (LR) or "low anxiety" (HR) groups based on their behavior in a variety of motor and behavioral tests. Both groups of animals were administered either FGF2 (5 ng/g) or vehicle by intraperitoneal injection every 48 hours for 3 weeks. One day after the last FGF2 injection, all animals were tested in the elevated plus-maze (EPM) and light-dark (LD) anxiety test.

The apparatus for the EPM test is constructed of black Plexiglass with four elevated open arms (70cm from the floor, 45cm long, and 12cm wide). Illumination is provided by a 40-watt desk lamp facing a wall and placed behind one of the closed arms. The scientists put the animal inside the system. Animals that are less anxious spend more time in the open arms whereas animals that are more anxious spend less time in the open arms.

The LD test is conducted in a 30 x 60 x 30-cm Plexiglas shuttle box with a translucent cover. Each box is divided into two equal-sized compartments by a wall with a 12 cm-wide open door. One compartment is painted white and brightly illuminated, and the other is painted black with very dim light. The time each rat spends in each compartment is monitored by rows of five photocells located 2.5 cm above the grid floor of each compartment. Animals that are less anxious spend more time in the light compartment.

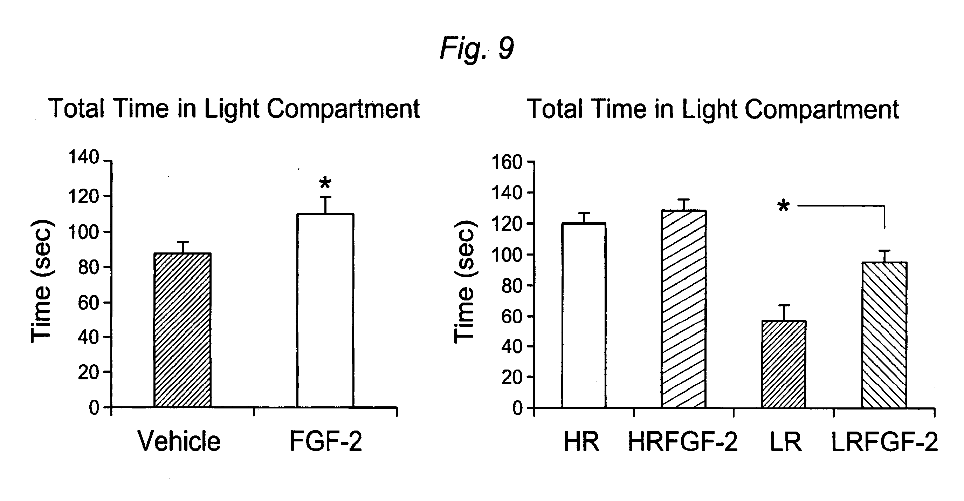

The results show that animals who received chronically administered FGF2 are less anxious than animals who receive vehicle (FIGURE 9, top). The anxiety-reducing effects of FGF2 are clearly more pronounced in rats who are inately more anxious (LR) prior to the FGF2 regimen (FIGURE 9, top).

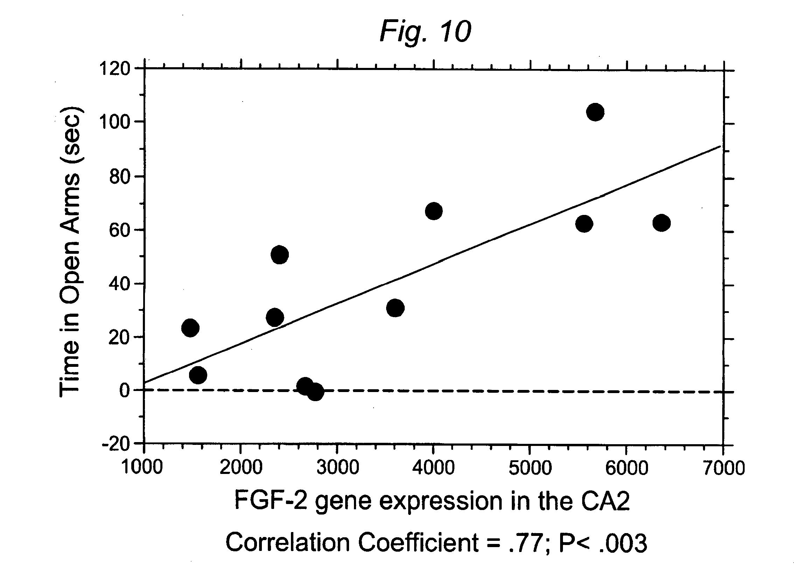

To further understand the relationship between FGF2 expression and anxiety, FGF2 expression was measured in the CA-2 region of the hippocampus of rat brains taken from rats which exhibited varying levels of anxiety (as measured by the EPM test). The results (FIGURE 10) show that FGF2 levels are inversely related to anxiety, *i.e.,* higher levels of inate FGF2 expression in rats correlate with lower levels of anxiety.

Taken as a whole, the Example shows that chronic FGF2 administration is useful for alleviating symptoms of anxiety in anxious animals and in subjects who are suffering from disorders such as MDD which are associated with anxiety. The data also shows that detection of FGF2 levels is useful for diagnosing anxiety or characterizing disorders associated with anxiety, such as Major Depression Disorder.

### Example 6: Differential Regulation of FGFR Splice Variants Associated with Chronic Stress

In the adult CNS, fibroblast growth factor receptor 2 (FGFR2) and fibroblast growth factor receptor 3 (FGFR3) are differentially distributed. The mRNA of these receptors undergoes alternative splicing in the exons coding for the carboxyl terminus of the Ig-like domain III. This mutually exclusive mRNA splicing produces two isoforms of FGFR2 and FGFR3 with significantly different ligand binding profiles: one isoform expressing exon IIIb (FGFR2b/FGFR3b), and one isoform expressing exon IIIc (FGFR2c/FGFR3c). Exon selection is strictly tissue-dependent during development with exon IIIb expressed in epithelial lineages and exon IIIc expressed in mesenchymal lineages. Cell cycle-dependent IIIb to IIIc swiches, however, have been induced *in vitro* by the exogenous addition of FGF1 and FGF2. This Example shows that chronic stress induces a decrease in the FGFR2 exon IIIc:IIIb splice variant expression ratio.

**Animals.** Twenty-four male Sprague-Dawley rats weighing 220-250g were ordered from Charles River (Wilmington, MA) and remained undisturbed for one week to acclimatize to housing conditions. The animals were housed in pairs on a 12h light-dark cycle (lights on 6:00 A.M.) with food and water available *ad libitum.* All experiments were conducted in accordance with the NIH Guide for the Care and Use of Animals and the University Committee on the Use and Care of Animals.

**FGF2 injection treatments and chronic unpredictable stress (CUS) conditions.** Half of the rats (n = 12) were administered vehicle (0.1M PBS with 100 µg/mL bovine serum albumin), and the other half (n = 12) were administered human recombinant FGF2 dissolved in vehicle in 5 ng/g dosages (Sigma, St. Louis, MO) every 48 hours for three weeks. All treatments were injected intra-peritonealy. During the same three week period as the FGF2 treatments, the vehicle group was either handled (n = 6) or exposed to CUS (n = 6). Likewise, the FGF2 injected group was either handled (n = 6) or exposed to CUS (n = 6). The animals were exposed to the following chronic unpredictable stressors (described in Isgor et al. (2004)): ether (30s), cold (2h), noise (15m), isolation (24h), or restraint (2h). The stressors were randomized to avoid habituation; sessions occurred once each day in either the morning or afternoon. The 2x2 (condition by treatment) design divided the subjects into nonstressed/vehicle (NS/V), nonstressed/FGF2 injection (NS/F), stressed/vehicle (S/V), and stressed/FGF2 injection (S/F) groups.

**Forced swim testing.** To test for possible FGF2 injection effects on anti-depressant behavior for other studies, all animals were subjected to forced swim testing according to Lucki (1997) 24h after the termination of the FGF2 treatments and CUS conditions.

**Brains.** The rats were sacrificed by the faculty by decapitation three days after termination of the forced swim testing. Brains were then removed and snap frozen in isopentane at -80°C.

**Total RNA extraction.** Gross dissections were performed on the frontal cortex of all brains. Total RNA extraction was executed following the reagent manufacturers' instructions. Tissues were homogenized in TRIzol (Invitrogen, Carlsbad, CA; monophasic phenol and guanidine isothiocyanate). Phase separation and RNA precipitation were carried out with chloroform and 2-propanol followed by centrifugation. RNA samples were purified using the RNeasy Mini Kit (Qiagen, Valencia, CA), repeatedly washing samples through spin columns. Pure RNA samples were reconstituted with RNase-free water, followed by an integrity analysis for 28s/18s ribosomal RNA peaks with the 2100 Bioanalyzer and LabChip (Agilent Technologies, Palo Alto, CA) system. Using the Bioanalyzer's concentration readings, the samples were normalized to 25 ng/µL of total RNA per subject and stored at-80°C.

**cDNA synthesis.** cDNA was synthesized using the iScript cDNA Synthesis Kit (Bio-Rad, Hercules, CA). All 24 RNA samples were reversed transcribed with iScript Reverse Transcriptase (Bio-Rad) and primed with oligo(dT) and random hexamer primers. Reaction mixes were incubated in an iCycler PCR unit (Bio-Rad) according to the manufacturer's standard 40min protocol. The double-stranded cDNA solutions were then analyzed for quality and concentration using Invitrogen's Quant-iT PicoGreen dsDNA kit. 10-fold serial dilutions were prepared with 2 µg/mL (high range) and 50 ng/mL (low range) stock DNA to generate standard curves. The fluorescently labeled samples were analyzed with a 1420 Victor² Multilabel Counter (EG&G Wallac, Wellesley, MA) using the basic Fluorescein protocol. Total cDNA samples were further normalized to fit the linear regression of the high range standard curve.

**Real time RT-PCR primer design.** Sequences for rat FGFR2 and FGFR3 exons were obtained from NCBI's Entrez Gene database (www.ncbi.nlm.nih.gov) and the Ensembl-Rat gene database (www.ensembl.org). Exon sequences were analyzed using NCBI's nucleotide and protein BLAST and were matched with their corresponding protein products within the receptor structures (FIGURE 11). FGFR2 and FGFR3 exon sequences were analyzed for secondary structure using the Mfold nucleic acid folding web server. Probable hairpin regions were noted for exclusion in primer design. Optimized primer sequences (FIGURE 11) were generated using the Primer3 web-based software. All primers were 18-22 base pairs, targeted amplicons of 75-150 bps, and purchased from Invitrogen's Custom Primer Synthesis service. The primer sequences (50 nmol/mL) were validated and tested for efficiency with 5-fold serial dilutions of pooled cDNA using iQ SYBR Green detection on an iCycler iQ Real Time RT-PCR unit (Bio-Rad).

**Real time RT-PCR quantification.** Real time reverse-transcriptase-PCR (RT-PCR) amplification reactions were performed to quantify relative abundances of mRNA (reverse transcribed to cDNA) of selected FGFR2 and R3 exons in each of the four treatment by condition groups (n=6). iQ SYBR Green Supermix detection was used on an iCycler iQ Real Time RT-PCR system (Bio-Rad) according to the manufacturer's recommendations, with the exception of preparing 19µL reactions instead of the instructed 50µL. Reference genes were omitted because total cDNA pools were normalized. Reactions were run in duplicates, increasing each group size to n=12. Two exons in juxtaposition were amplified per plate for relative comparison (emphasis placed on exon IIIb and exon IIIc). Reaction wells were arranged to equalize positional representation amongst all groups. PCR protocol was as follows: hot start at 95°C for 30s, followed by 40 cycles of denature at 95°C for 15s, annealing at 60°C for 15s, and elongation at 72°C for 15s. Florescence was quantified after every cycle, and melt curve analysis was performed following amplification to ensure single product reactions. Thorough methodology is described by Kerman et al. (2006).

**Data analysis** Real time RT-PCR data was output as threshold cycle (Ct) values, using Bio-Rad's iCycler iQ software's algorithm to calculate the optimum fluorescence thresholds for reliable detection (the mean florescence value of the first ten PCR cycles plus 10 standard deviations). Essentially, lower Ct values indicate higher amounts of initial target cDNA because fewer PCR cycles are required to reach fluorescence thresholds. Ct values for all reactions were then grouped and presented as means with standard errors. Sample sizes were 9-12 per group due to outlier (≥2 StDev from mean) exclusion. Mean fold changes were calculated using a 2^{ΔCt} method (modification of technique described by Livak and Schmittgen (2001)), comparing mean Ct values within one variable (treatment effects within one condition or condition effects within one treatment). This 2^{ΔCt} method assumes equal primer efficiencies; however, for the purposes of quantifying relative expression, it is acceptable if the primers are validated. Exon IIIc to exon IIIb ratios were determined by calculating individual 2^{ΔCt} values between corresponding reactions of the two exons. Ratios were sorted similarly to the Ct value groups and presented as mean ratios with standard errors. Statistical significance tests for differences in multiple exon mean Ct values and individual IIIc:IIIb ratios were performed using two-factor ANOVA and Student's *t-test* for each comparison variable. Significance level was set as p < 0.05. All statistical analysis was done in Microsoft Excel 2003.

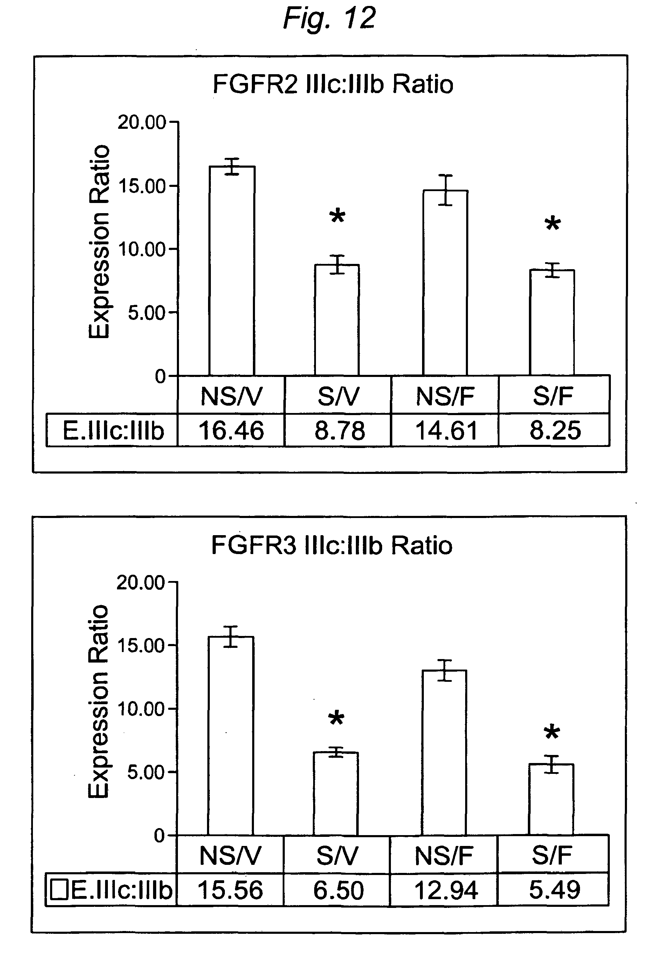

Results. Chronic stress induced significant decreases in the exon IIIc to exon IIIb (mutually exclusive expression) splice variant ratio in the vehicle group (P<0.00004) and in the FGF2 injection group (P<0.005) (FIGURE 12). While exon IIIc expression remained relatively higher than IIIb in all groups, IIIb expression increased significantly with stress while IIIc expression changed only slightly. Thus, stress increases expression of the IIIb variant relative to the IIIc variant for both FGFR2 and FGFR3. FGF2 injection did not alter the IIIc:IIIb ratio significantly in either the NS or S group (P>0.1), nor did it significantly affect the magnitude of IIIc:IIIb ratio changes caused by stress.

The results show that the affinity of FGFR2 or FGFR3 for endogenous ligands such as FGF2 and FGF9 (which are differentially expressed in MDD subjects) or for exogenous ligands *(e.g.,* pharmacological peptides or other compounds) can be altered by stress. The invention described herein provides methods of detecting variations in FGFR2 and FGFR3 splicing and modifying subject care accordingly. In another embodiment, the invention provides methods for identifying and optimizing therapeutics for treating depression and related ailments.

### Example 7: Differential Regulation of Genes in the Locus Coeruleus and the Dorsal Raphe in Subjects with Bipolar and Major Depression Disorder

The Locus Coeruleus (LC) and the Dorsal Raphe (DR) are the major sources for noradrenergic and serotonergic innervation of the brain respectively. Dysregulation of these neurotransmitters has been implicated in psychiatric disorders. This Example uses postmortem brains of patients with MDD (N=12), BPD (N=6), and healthy subjects (N=9) to contrast gene expression profiles in the LC and DR regions of their brains. All subjects met inclusion criteria of brain pH > 6.6 and zero agonal factors. Total RNA samples from laser capture microdissected LC and DR samples were extracted, amplified, and probed with Affymetrix high density oligonucleotide microarrays. Gene expression data were analyzed by ANOVA of robust multichip average algorithm (p≤ 0.1) and by MAS5.0 "present" call algorithm (min.of 50% presences in one of the 3 health states). Genes meeting these criteria were analyzed using Ingenuity Pathway Analysis (IPA). Compared to healthy individuals, 774 and 636 genes show altered expression in the LC and 627 and 656 genes show altered expression in the DR of MDD and BPD patients, respectively.

**LC gene expression patterns:** The data is summarized in Table 3. Ingenuity Pathway Analysis revealed that 10 genes of the glutamate receptor signaling pathway are significantly altered in MDD (p<0.01) but not in BPD. Glutamate signaling gene expression alterations are present in following synaptic compartments of the locus coeruleus: glial cells, presynpatic neurons, and postsynaptic neurons. This shows that glutamate signaling is altered in LC of MDD patients. Glial transporters, glutamine synthetase, AMPA, kainate, GRM1 and GRM7 are thus targets for treating glutamatergic imbalance.

The expression of genes related to growth, *i*.*e*., fibroblast growth factors, are also significantly altered in the LC of MDD patients. Drugs that target FGFR3, TrkB receptor, growth hormone receptor, or which mimic the actions of FGF-2, would increase neurite outgrowth in the LC and reserve neuronal loss.

Genes that are almost exclusively expressed in glia are significantly downregulated. These genes are useful markers for global glial alterations in MDD patients.

**DR gene expression patterns.** The data is summarized in Table 4. IPA analyses of the DR revealed alterations in the expression of a number of genes in growth factor-related pathways in MDD. Expression of most of the altered genes was downregulated. Likewise, the expression of a number of genes in growth factor-related pathways was downregulated in samples from the BP cohort. However, these genes were distinct from those detected in the MDD cohort. Several genes that were common to both disorders were identified; their expression was altered in the same direction in MDD and BPD subjects.

### Example 8. Anxiolytic Effect of FGF9

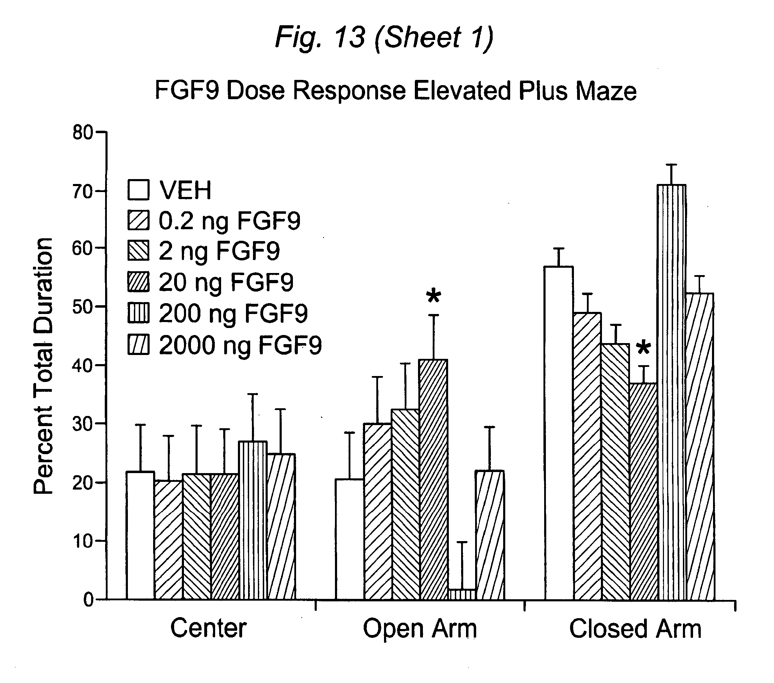

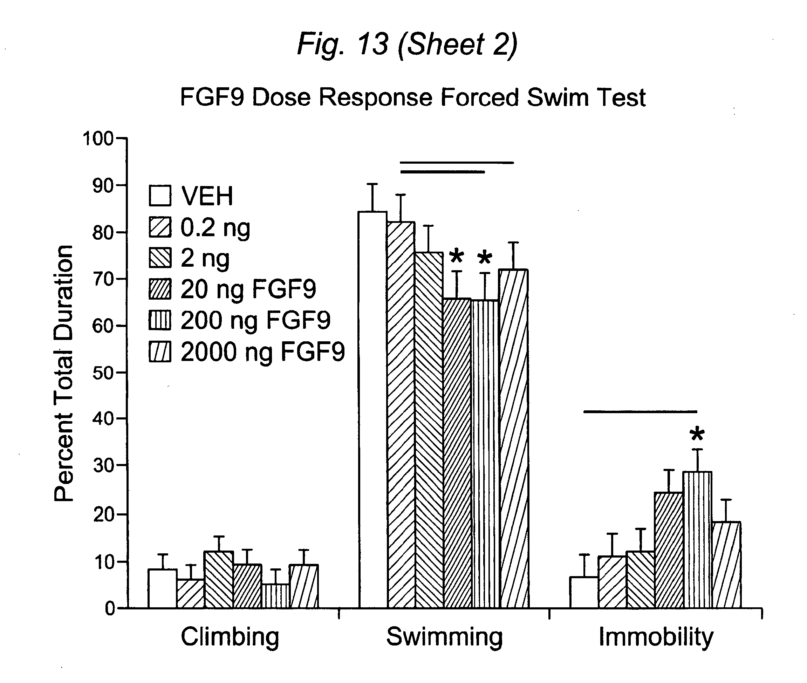

Rats were implanted with guide cannulae into the left ventricle. Adult male Sprague-Dawley rats were microinjected (intracerebroventricularly (icv)) with either vehicle or FGF9 at the concentrations indicated in FIGURE 13. Total volumes (8 µl) were infused at 1.0 µl/min and an injection syringe was held in place for 3 min to allow for diffusion. Fifteen minutes after microinjection, anxiety was tested in the rats using the elevated plus maze ("EPM"). Rats were placed in the center of the EPM and allowed to explore freely under dim light for 5 min. The animals were evaluated for time spent in the center, open arms, and closed arms of the maze, where increased time in the open arms versus the closed arm correlates with less anxiety. Twenty-four hours after microinjection, depression was evaluated in the rats using the forced swim test (FST).

The data show that acute administration of FGF9 at 2 to 2000 ng successfully decreased anxiety-related behavior using this animal model system.

### Example 9. Effect of Chronic Administration of FGF9

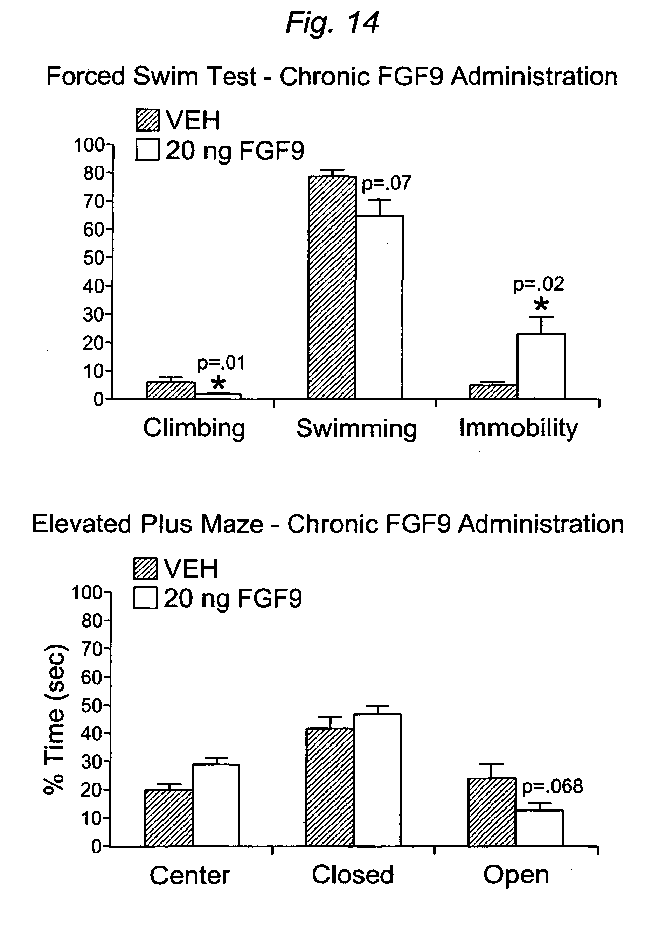

Animals were injected icv daily for 3 weeks with 20 ng FGF9. All behavioral testing was performed in the lights on portion of the light/dark cycle. FIGURE 14 shows that chronic FGF9 administration increased the depression-like effect seen in the forced swim test compared to acute administration. Chronic FGF9 had an anxiogenic effect on the elevated plus maze task, which is opposite to the anxiolytic effect seen with acute administration.

### Example 10. Effect of siRNAs Targeting FGF 2 or FGF9

Small interfering RNAs (siRNAs) targeting either FGF2 or FGF9 expression were designed and validated. COS7 cells were transfected with a plasmid vector (psiCHECK-2, Promega) containing either FGF2 or FGF9 and co-transfected with either FGF2 or FGF9 siRNA. The transfection efficiency and the ability of siRNA targeting FGF2 or FGF9 to decrease protein levels were evaluated by a dual-luciferase reporter assay. The effects of FGF2 siRNA inhibition on FGF2 expression was determined in a human gliomal cell line (U87MG) that endogenously expresses high levels of FGF2. Sequences have been identified that are effective (>80% knockdown) and *specific in vitro.*

| **siRNA** | **Sense Strand (5' -> 3') (SEQ ID NO: 36)** | **Antisense Strand (5' → 3') (SEQ ID NO: 37)** |
|---|---|---|
| **FGF9** | UGGAUGAGGACUUGCCGAUUU | AUCGGCAAGUCCUCAUCCAUU |

| | | |
|---|---|---|
| The "UU" nucleotides at the 3' end of each strand correspond to 3' overhangs, but the sense and/or antisense strand may alternatively comprise "dTdT" 3' overhangs or 3' overhangs that have complementarity to the target sequence or the complementary strand thereof. | | |

**TABLE 1A**

| **Genes associated with suicide.** | | | | | | | | | | |
|---|---|---|---|---|---|---|---|---|---|---|
| **Symbol** | **Suicide dysregulation** | **Name** | **Best Accession** | **Cytoband** | **Anterior Cingulate Fold Change** | | **Amygdala Fold Change** | | **Cerebellum Fold Change** | |
| | | | | | **Suicide Vs Mood** | **Suicide Vs Control** | **Suicide Vs Mood** | **Suicide Vs Control** | **Suicide Vs Mood** | **Suicide Vs Control** |
| FOS | 3 region | V-fos FBJ murine osteosarcoma viral oncogene homolog | BM894421 | 14q24.3 | **0.671** | **0.679** | **0.716** | **0.638** | **0.770** | **0.659** |
| CAPN3 | 2 region | "Calpain 3, (p94)" | BC003169 | 15q15.1-q21.1 | **0.783** | **0.799** | **0.567** | 0.651 | 0.846 | 0.926 |
| FOS | 2 region | V-fos FBJ murine osteosarcoma viral oncogene homolog | BM894421 | 14q24.3 | **0.671** | **0.679** | **0.716** | **0.638** | **0.770** | **0.659** |
| CAPN3 | ancng | "Calpain 3, (p94)" | BC003169 | 15q15.1-q21.1 | **0.783** | **0.799** | **0.567** | 0.651 | 0.846 | 0.926 |
| FGF12 | ancng | Fibroblast growth factor 12 | BC022524 | 3q28 | **1.393** | **1.303** | 1.060 | 1.167 | 0.896 | 1.074 |
| FOS | ancng | V-fos FBJ murine osteosarcoma viral oncogene homolog | BM894421 | 14q24.3 | **0.671** | **0.679** | **0.716** | **0.638** | **0.770** | **0.659** |
| MAP2 | ancng | Microtubule-associated protein 2 | AL535786 | 2q34-q35 | **1.389** | 1.303 | 0.914 | 1.084 | 0.921 | 0.999 |
| LRIG1 | ancng | Leucine-rich repeats and immunoglobulin-like domains 1 | AI459030 | 3p14 | **0.799** | 0.689 | 1.008 | 0.897 | **0.965** | 0.810 |
| TENS1 | ancng | Tensin-like SH2 domain containing 1 | CN480168 | 7p13-p12.3 | **0.704** | 0.737 | 0.992 | 0.854 | **0.953** | 0.721 |
| ADAMTS1 | ancng | "A disintegrin-like and metalloprotease (reprolysin type) with thrombospondin type 1 motif, 1" | CA446773 | 21q21.2 | **0.796** | 0.721 | **0.941** | 0.649 | **1.014** | 0.822 |
| CNTN1 | ancng | Contactin 1 | U07819 | 12q11-q12 | **1.320** | 1.283 | 1.038 | 1.098 | 0.883 | 0.994 |
| GABRG2 | ancng | "Gamma-aminobutyric acid (GABA) A receptor, gamma 2" | BI754570 | 5q31.1-q33.1 | **1.376** | 1.282 | **0.902** | 1.236 | 0.858 | 1.028 |
| EFEMP1 | ancng | EGF-containing fibulin-like extracellular matrix protein 1 | CR611721 | 2p16 | **0.791** | **0.749** | **0.784** | 0.826 | **0.979** | 0.787 |
| TPBG | ancng | Trophoblast glycoprotein | CA413562 | 6q14-q15 | **1.495** | 1.334 | **1.057** | 1.323 | 1.029 | 1.025 |
| C3orf4 | amygdala | Chromosome 3 open reading frame 4 | CD102473 | 3p11-q11 | 0.897 | **0.909** | **0.705** | 0.763 | 0.903 | 0.921 |
| EVI2A | amygdala | Ecotropic viral integration site 2A | CA415486 | 17q11.2 | 0.883 | **0.862** | **0.669** | 0.714 | 0.899 | 0.884 |
| CD9 | amygdala | CD9 antigen (p24) | AW864408 | 12p13.3 | 0.940 | **0.850** | **0.760** | **0.798** | **0.821** | **0.702** |
| MOG | amygdala | Myelin oligodendrocyte glycoprotein | NM_002433 | 6p22.1 | **0.847** | **0.792** | **0.644** | 0.711 | 0.905 | 0.883 |
| CAPN3 | amygdala | "Calpain 3, (p94)" | BC003169 | 15q15.1-q21.1 | **0.783** | **0.799** | **0.567** | 0.651 | 0.846 | 0.926 |
| UGT8 | amygdala | UDP glycosyltransferase 8 (UDP-galactose ceramide galactosyltransferase) | CN275924 | 4q26 | **0.945** | **0.781** | **0.710** | 0.776 | 0.926 | 0.947 |
| ASPA | amygdala | "Aspartoacylase (aminoacylase 2, Canavan disease)" | BF115120 | 17pter-p13 | **0.903** | **0.816** | **0.761** | 0.763 | 0.910 | 0.923 |
| PLP1 | amygdala | "Proteolipid protein 1 (Pelizaeus-Merzbacher disease, spastic paraplegia 2, uncomplicated)" | BP194315 | Xq22 | **0.935** | **0.888** | **0.765** | 0.751 | 0.876 | 0.848 |
| ENPP2 | amygdala | Ectonucleotide pyrophosphatase/phosphodiestera se 2 (autotaxin) | CR606785 | 8q24.1 | **0.816** | **0.817** | **0.602** | **0.717** | 0.813 | **0.851** |
| ENPP2 | amygdala | Ectonucleotide pyrophosphatase/phosphodiestera se 2 (autotaxin) | CR606785 | 8q24.1 | **0.816** | **0.817** | **0.602** | **0.717** | 0.813 | **0.851** |
| RPH3A | amygdala | Rabphilin 3A homolog (mouse) | CR613722 | 12q24.13 | 1.241 | **1.176** | **1.259** | 1.283 | 0.994 | 1.095 |
| SLC31A2 | amygdala | "Solute carrier family 31 (copper transporters), member 2" | NM_001860 | 9q31-q32 | 0.875 | **0.864** | **0.707** | 0.798 | 0.957 | 0.966 |
| FOS | amygdala | V-fos FBJ murine osteosarcoma viral oncogene homolog | BM894421 | 14q24.3 | **0.671** | **0.679** | **0.716** | **0.638** | **0.770** | **0.659** |
| CNP | amygdala | "2',3'-cyclic nucleotide 3' phosphodiesterase" | CD369491 | 17q21 | 0.836 | **0.838** | **0.754** | 0.761 | 0.910 | 0.872 |
| SEPP1 | amygdala | "Selenoprotein P, plasma, 1" | BF941781 | 5q31 | **0.867** | **0.771** | **0.733** | 0.785 | **0.842** | 0.667 |
| PMP22 | amygdala | Peripheral myelin protein 22 | BG939651 | 17p12-p11.2 | **0.845** | **0.772** | **0.793** | 0.793 | **0.927** | 0.779 |
| GREM1 | amygdala | "Gremlin 1 homolog, cysteine knot superfamily (Xenopus laevis)" | NM_013372 | 15q13-q15 | 0.827 | **0.912** | **0.800** | 0.798 | 0.970 | 0.991 |
| GPR37 | amygdala | G protein-coupled receptor 37 (endothelin receptor type B-like) | BF966147 | 7q31 | 0.991 | **0.878** | **0.735** | 0.784 | 0.954 | 0.917 |
| HSPA2 | amygdala | Heat shock 70kDa protein 2 | BM979297 | 14q24.1 | 1.008 | **0.851** | **0.702** | 0.724 | 0.908 | 0.883 |
| CD74 | amygdala | "CD74 antigen (invariant polypeptide of major histocompatibility complex, class II antigen-associated)" | BI262425 | 5q32 | **0.806** | **0.766** | **0.787** | 0.746 | **0.895** | 0.738 |
| FA2H | amygdala | Fatty acid 2-hydroxylase | AA454978 | 16q23 | 0.898 | **0.875** | **0.685** | 0.734 | 0.906 | 0.922 |
| C20orf35 | amygdala | Chromosome 20 open reading frame 35 | BI711408 | 20q13.12 | 0.865 | **0.902** | **0.759** | 0.750 | 0.930 | 0.868 |
| ZFYVE16 | amygdala | "Zinc finger, FYVE domain containing 16" | AK127003 | 5p15.2-q14.3 | **0.862** | **0.797** | **0.772** | 0.776 | 0.868 | 0.992 |
| MAG | amygdala | Myelin associated glycoprotein | X98405 | 19q13.1 | **0.870** | **0.829** | **0.685** | 0.726 | 1.011 | 0.904 |
| SATB2 | amygdala | SATB family member 2 | AW970253 | 2q33 | 1.231 | **1.188** | **1.549** | 1.302 | 0.975 | 1.019 |
| TF | amygdala | Transferrin | CB156966 | 3q22.1 | **0.890** | **0.829** | **0.669** | 0.711 | 0.878 | 0.881 |
| OPN3 | amygdala | "Opsin 3 (encephalopsin, panopsin)" | BU631266 | 1q43 | 1.257 | **1.151** | **1.293** | 1.343 | 1.081 | 1.044 |
| CGI-38 | amygdala | Brain specific protein | BM981844 | 16q22.1 | 1.000 | **1.000** | **1.667** | 1.563 | 1.000 | 1.000 |
| ST18 | amygdala | Suppression of tumorigenicity 18 (breast carcinoma) (zinc finger protein) | AI741795 | 8q11.23 | **0.875** | **0.804** | **0.652** | 0.711 | 0.983 | 0.945 |
| RNASE1 | amygdala | "Ribonuclease, RNase A family, 1 (pancreatic)" | AL046791 | 14q11.2 | 0.937 | **0.932** | **0.760** | 0.771 | 0.962 | 0.967 |
| KLK6 | amygdala | "Kallikrein 6 (neurosin, zyme)" | CA945202 | 19q13.3 | 0.882 | **0.878** | **0.739** | 0.781 | 0.930 | 0.973 |
| ZNF536 | amygdala | Zinc finger protein 536 | AK091043 | 19q12 | | | **1.584** | 1.603 | | |
| CX3CR1 | amygdala | Chemokine (C-X3-C motif) receptor 1 (CX3CR1) | CB047889 | 3p21 | 0.861 | **0.808** | 0.705 | 0.779 | 0.885 | 0.837 |
| CHL1 | cblm | Cell adhesion molecule with homology to L1CAM (close homolog of L1) | BF966608 | 3p26.1 | 1.119 | 1.088 | **0.978** | **1.297** | **0.786** | **0.786** |
| ZBTB16 | cblm | Zinc finger and BTB domain containing 16 | Z19002 | 11q23.1 | **1.231** | 1.235 | 1.199 | **1.161** | **1.446** | **1.625** |
| FOS | cblm | V-fos FBJ murine osteosarcoma viral oncogene homolog | BM894421 | 14q24.3 | **0.671** | 0.679 | 0.716 | **0.638** | **0.770** | **0.659** |
| HLA-DPA1 | cblm | "Major histocompatibility complex, class II, DP alpha 1" | AI554919 | 6p21.3 | 0.874 | **0.860** | 0.831 | **0.839** | **0.771** | **0.799** |

**TABLE 1B**

| **Symbol** | **Suicide dysregulation** | **Name** | **Best Accession** | ***C*ytoband** | **Anterior Cingulate Fold Change** | |
|---|---|---|---|---|---|---|
| | | | | | **Suicidal MDD Vs Non-suicidal MDD** | ***S*uicidal MDD Vs Con**t**rol** |
| PRPS2 | ancng | Phosphoribosyl pyrophosphate synthetase 2 | NM_002765 | Xp22.3-p22.2 | **1.287** | **1.251** |
| FGF9 | ancng | Fibroblast growth factor 9 (glia-activating factor) | D14838 | 13q11-q12 | **1.270** | **1.288** |
| RIMS2 | ancng | Regulating synaptic membrane exocytosis 2 | BC043144 | 8q22.3 | **1.243** | **1.288** |
| TM4SF9 | ancng | Tetraspanin 5 | BX649011 | 4q23 | **1.488** | **1.358** |
| PCLO | ancng | Piccolo (presynaptic cytomatrix protein) | AB011131 | ^{.}7q11.23-q21.3 | **1.555** | **1.383** |
| GNB5 | ancng | Guanine nucleotide binding protein (G protein), beta 5 | AK092059 | 15q21.2 | **1.206** | **1.263** |
| GABRA1 | ancng | Gamma-aminobutyric acid (GABA) A receptor, alpha 1 | NM_000806 | 5q34-q35 | **1.353** | **1.356** |
| NLK | ancng | Nemo like kinase | BC064663 | 17q11.2 | **1.413** | **1.351** |
| CDH18 | ancng | Cadherin 18, type 2 | B031051 | 5p15.2-p15.1 | **1.217** | **1.260** |
| NR1D2 | ancng | Nuclear receptor subfamily 1, group D, member 2 | AB209091 | 3p24.2 | **1.476** | **1.319** |
| R3HDM | ancng | R3H domain containing 1 | BX538168 | 2q21.3 | **1.208** | **1.222** |
| COL5A2 | ancng | Collagen, type V, alpha 2 | NM_000393 | 2q14-q32 | **1.209** | **1.226** |
| | ancng | Cerebellar mRNA | AK126654 | | **1.229** | **1.303** |
| NRXN1 | ancng | Neurexin 1 | AB011150 | 2p16.3 | **1.247** | **1.230** |
| NCALD | ancng | Neurocalcin delta | AB209015 | 8q22-q23 | **1.284** | **1.306** |
| CADPS2 | ancng | Ca2+-dependent activator protein for secretion 2 | NM_017954 | | **1.393** | **1.426** |
| TRIM23 | ancng | Tripartite motif-containing 23 | NM_001656 | 5q12.3 | **1.231** | **1.227** |
| STAT4 | ancng | Signal transducer and activator of transcription 4 | NM_003151 | 2q32.2-q32.3 | **1.228** | **1.314** |
| HLA-A | ancng | Major histocompatibility complex, class I, A | AB209117 | 6p21.3 | **0.771** | **0.788** |
| LYPDC1 | ancng | LY6/PLAUR domain containing 1 | AK122643 | 2q21.2 | **0.819** | **0.787** |
| RYR1 | ancng | Ryanodine receptor 1 (skeletal) | NM_000540 | 19q13.1 | **0.749** | **0.791** |

**TABLE 1C Genes associated with substance abuse comorbidity.**

| **Symbol** | **Suicide dysregulation** | **Name** | **Best Accession** | **Cytoband** | **Anterior Cingulate Fold Change** | |
|---|---|---|---|---|---|---|
| | | | | | **Substance Abuse MDD Vs Non-S.A. MDD** | **Substance Abuse MDD Vs Control** |
| NS3TP1 | ancng | HCV NS3-transactivated protein 1 | AK000759 | 2p24.3-q21.3 | **1.280** | **1.230** |
| DZIP1 | ancng | DAZ interacting protein 1 | NM_198968 | 13q32.1 | **1.259** | **1.247** |
| STMN4 | ancng | Stathmin-like 4 | NM_030795 | 8p21.2 | **1.255** | **1.217** |
| CDH18 | ancng | Cadherin 18, type 2 | BC031051 | 5p15.2-p15.1 | **1.269** | **1.238** |
| KCMF1 | ancng | Potassium channel modulatory factor 1 | NM_020122 | 2p11.2 | **1.230** | **1.214** |
| CRYZL1 | ancng | Crystallin, zeta (quinone reductase)-like 1 | AK057604 | 21q21.3 | **1.461** | **1.341** |
| OSBPL8 | ancng | Oxysterol binding protein-like 8 | NM_020841 | 12q14 | **1.551** | **1.403** |
| FGF14 | ancng | Fibroblast growth factor 14 | AY188178 | 13q34 | **1.291** | **1.231** |
| HSPCB | ancng | Heat shock 90kDa protein 1, beta | AY359878 | 6p12 | **1.264** | **1.210** |
| DDX50 | ancng | DEAD (Asp-Glu-Ala-Asp) box polypeptide 50 | BC000272 | 10q22.1 | **1.338** | **1.219** |
| GNAZ | ancng | G protein, alpha z polypeptide | BC037333 | 22q11.22 | **1.279** | **1.210** |
| CACNB2 | ancng | Calcium channel, voltage-dependent, B2 | AB208917 | 10p12 | **1.270** | **1.263** |
| RASL11B | ancng | RAS-like, family 11, member B | BC025694 | 4q12 | **1.272** | **1.216** |
| DNM2 | ancng | Dynamin 2 | AK127033 | 19p13.2 | **0.805** | **0.813** |
| THG-1 | ancng | TSC22 domain family, member 4 | NM_030935 | 7p21-p15 | **0.829** | **0.785** |

**TABLE 2A**

| **Lithium-responsive genes known in literature: confirmed in non-human primates** | | | |
|---|---|---|---|
| **Symbol** | **UGCluster** | **Cytoband** | **Name** |
| AP1S1 | NM_057089 | 7q22.1 | adaptor-related protein complex 1, sigma 1 subunit |
| CLOCK | NM_004898 | 4q12 | clock homolog (mouse) |
| FGF8 | NM_033163-5 | 10q24 | fibroblast growth factor 8 (androgen-induced) |
| GSK3B | NM_002093 | 3q13.3 | glycogen synthase kinase 3 beta |
| NFKB1 | NM_003998 | 4q24 | nuclear factor of kappa light polypeptide gene enhancer in B-cells 1 (p105) |

**TABLE 2B**

| **Novel genes that are both lithium-responsive and dysregulated in BPD** | | | |
|---|---|---|---|
| **Symbol** | **UGCluster** | **Cytoband** | **Name** |
| A2M | Hs.212838 | 12p13.3-p12.3 | Alpha-2-macroglobulin |
| AP1S1 | Hs.489365 | 7q22.1 | Adaptor-related protein complex 1, sigma 1 subunit |
| BRD7 | In multiple clusters | 16q12 | bromodomain containing 7 |
| CA10 | Hs.463466 | 17q21 | Carbonic anhydrase X |
| CD74 | Hs.436568 | 5q32 | CD74 antigen (invariant polypeptide of major histocompatibility complex, class II antigen-associated) |
| CDK5R2 | Hs.158460 | 2q35 | Cyclin-dependent kinase 5, regulatory subunit 2 (p39) |
| CNN2 | Hs.169718 | 21q11.1 | Calponin 2 |
| DSTN | Hs.304192 | 20p12.1 | Destrin (actin depolymerizing factor) |
| ELAVL4 | Hs.213050 | 1p34 | ELAV (embryonic lethal, abnormal vision, Drosophila)-like 4 (Hu antigen D) |
| FLJ10094 | Hs.128258 | 13q14.11 | Hypothetical protein FLJ10094 |
| HNRPH2 | Hs.278857 | Xq22 | Heterogeneous nuclear ribonucleoprotein H2 (H') |
| IGFBP6 | Hs.274313 | 12q13 | Insulin-like growth factor binding protein 6 |
| MOGAT2 | Hs.288568 | 11q13.5 | Monoacylglycerol O-acyltransferase 2 |
| MYLK | Hs.477375 | 3q21 | Myosin, light polypeptide kinase |
| NRIP1 | Hs.155017 | 21q11.2 | Nuclear receptor interacting protein 1 |
| PSG5 | Hs.558372 | 19q13.2 | Pregnancy specific beta-1-glycoprotein 5 |
| PTP4A2 | In multiple clusters | 1p35 | Protein-tyrosine phosphatase, type 4A, 2P4A2 |
| RPLP2 | Hs.437594 | 11p15.5-p15.4 | Ribosomal protein, large, P2 |
| RPS14 | Hs.381126 | 5q31-q33 | Ribosomal protein S14 |
| RPS9 | In multiple clusters | 19q13.4 | Ribosomal protein S9 |
| SST | Hs.12409 | 3q28 | Somatostatin |
| UBQLN2 | Hs.522668 | Xp11.23-p11.1 | Ubiquilin 2 |
| UXT | Hs.172791 | Xp11.23-p11.22 | Ubiquitously-expressed transcript |

**TABLE 3**

| **Summary of LC dysregulated genes in subjects with major depression disorder** | | | |
|---|---|---|---|
| **RefSequ #** | **Name** | **p (C vs.MD)** | **FC (fold change) MD vs. C** |
| **A. Glutamate signaling** | | | |
| NM_004171 | SLC1A2 (glial high affinity glutamate transporter) | 0.04 | -1.26 |
| NM_004172 | SLC1A3 (glial high affinity glutamate transporter) | 0.03 | -1.59 |
| NM_002065 | GS (glutamine synthetase) | 0.02 | -1.28 |
| NM_020346 | VGlutT2 | 0.01 | 1.31 |
| NM_005271 | Glutamate dehydrogenase | 0.05 | -1.27 |
| NM_181875 | mGlutR7 (transcript variant 3) | 0.07 | -1.06 |
| NM_000827 | AMPA1 | 0.09 | 1.14 |
| NM_000828 | AMPA3 | 0.01 | 1.16 |
| NM_175611 | GRIK1 | 0.02 | 1.22 |
| NM_000838 | mGlutR1 | 0.05 | 1.08 |
| NM_000842 | mGlutR5 | 0.01 | 1.16 |

| **B. Growth factors and growth related gene** | | | |
|---|---|---|---|
| NM_002006 | FGF2 | 0.10 | -1.07 |
| NM_002010 | FGF9 | 0.02 | 1.16 |
| NM_000142 | FGFR3 | 0.04 | -1.17 |
| NM_001007097 | TrkB receptor | 0.03 | -1.45 |
| NM_000163 | Growth hormone receptor | 0.10 | -1.20 |

| **C. Glial markers** | | | |
|---|---|---|---|
| NM_002055 | Glial fibrillary acidic protein | 0.05 | -1.32 |
| NM_000165 | Gap junction protein, alpha 1, 43kDa (connexin 43) | 0.04 | -1.86 |
| NM_006783 | Gap junction protein, beta 6 (connexin 30) | 0.02 | -1.93 |
| NM_080388 | S100 calcium binding protein A16 | 0.02 | -1.15 |

**TABLE 4**

| Summary of dysregulated genes in DR of MDD and BPD subjects | | | | | |
|---|---|---|---|---|---|
| **MDD genes** | | | | | |
| **RefSeq8_ID** | **Description** | **FC MD vs. C** | **p value MD vs. C** | **FC BP vs. C** | **p value BP vs. C** |
| NM_001709 | brain-derived neurotrophic factor (BDNF), transcript variant 4, mRNA | -1.207 | 0.052 | -1.164 | 0.140 |
| NM_003670 | basic helix-loop-helix domain containing, class B, 2 (BHLHB2), mRNA | -1.185 | 0.017 | -1.175 | 0.160 |
| NM_001200 | bone morphogenetic protein 2 (BMP2), mRNA | -1.059 | 0.085 | -1.035 | 0.151 |
| NM_0010085 40 | chemokine (C-X-C motif) receptor 4 (CXCR4), transcript variant 1, mRNA | -1.154 | 0.001 | -1.067 | 0.260 |
| NM_145793 | GDNF family receptor alpha 1 (GFRA1), transcript variant 2, mRNA | -1.062 | 0.067 | -1.020 | 0.429 |
| NM_013372 | gremlin 1, cysteine knot superfamily, homolog (Xenopus laevis) (GREM1), mRNA | -1.153 | 0.020 | -1.099 | 0.194 |
| NM_002253 | kinase insert domain receptor (a type III receptor tyrosine kinase) (KDR), mRNA | -1.081 | 0.092 | -1.065 | 0.362 |
| NM_005924 | mesenchyme homeobox 2 (MEOX2), mRNA | -1.105 | 0.028 | -1.045 | 0.417 |
| NM_002530 | neurotrophic tyrosine kinase, receptor, type 3 (NTRK3), transcript variant 2, mRNA | 1.080 | 0.072 | 1.090 | 0.165 |
| NM_002660 | phospholipase C, gamma 1 (PLCG1), transcript variant 1, mRNA | 1.165 | 0.098 | 1.159 | 0.122 |
| NM_005904 | SMAD, mothers against DPP homolog 7 (Drosophila) (SMAD7), mRNA | -1.195 | 0.056 | -1.037 | 0.439 |
| NM_000346 | SRY (sex determining region Y)-box 9 (campomelic dysplasia, autosomal sex-reversal) (SOX9), mRNA | -1.204 | 0.093 | -1.128 | 0.251 |
| NM_005749 | transducer of ERBB2, 1 (TOB1), mRNA | -1.946 | 0.034 | -1.866 | 0.113 |
| NM_005429 | vascular endothelial growth factor C (VEGFC), mRNA | -1.150 | 0.034 | -1.099 | 0.168 |
| NM_000756 | corticotropin releasing hormone (CRH), mRNA | 1.205 | 0.021 | -1.032 | 0.146 |
| | | | | | |

| **BPD genes** | | | | | |
|---|---|---|---|---|---|
| **RefSeq8_ID** | **Description** | **FC MD vs. C** | **p value MD vs. C** | **FC BP vs. C** | **p value BP vs. C** |
| NM_004281 | BCL2-associated athanogene 3 mRNA | -1.207 | 0.170 | -1.345 | 0.073 |
| NM_203330 | CD59 molecule, complement regulatory protein (CD59), transcript variant 1, mRNA | -1.040 | 0.326 | -1.107 | 0.042 |
| NM_000089 | collagen, type I, alpha 2 (COL1A2), mRNA | -1.034 | 0.325 | -1.134 | 0.049 |
| NM_001331 | catenin (cadherin-associated protein), delta 1 (CTNND1), mRNA | 1.049 | 0.075 | 1.083 | 0.011 |
| NM_001963 | epidermal growth factor (beta-urogastrone) (EGF), mRNA | -1.048 | 0.149 | -1.083 | 0.030 |
| NM_004508 | isopentenyl-diphosphate delta isomerase 1 (IDI1), mRNA | -1.168 | 0.192 | -1.246 | 0.069 |
| NM_058004 | phosphatidylinositol 4-kinase, catalytic, alpha polypeptide (PIK4CA), transcript variant 2, mRNA | -1.071 | 0.283 | -1.235 | 0.025 |
| NM_016224 | sorting nexin 9 (SNX9), mRNA | -1.105 | 0.134 | -1.162 | 0.085 |
| NM_003239 | transforming growth factor, beta 3 (TGFB3), mRNA | 1.022 | 0.364 | 1.204 | 0.026 |
| NM_133646 | sterile alpha motif and leucine zipper containing kinase AZK (ZAK), transcript variant 2, mRNA | -1.111 | 0.159 | -1.208 | 0.094 |
| NM_000898 | monoamine oxidase B (MAOB), nuclear gene encoding mitochondrial protein, mRNA | -1.083 | 0.156 | -1.102 | 0.042 |

| **Common genes** | | | | | |
|---|---|---|---|---|---|
| **RefSeq8_ID** | **Description** | **FC MD vs. C** | **p value MD vs. C** | **FC BP vs. C** | **p value BP vs. C** |
| NM_004723 | rho/rac guanine nucleotide exchange factor (GEF) 2 (ARHGEF2), mRNA | 1.089 | 0.099 | 1.073 | 0.074 |
| NM_078467 | cyclin-dependent kinase inhibitor 1A (p21, Cip1) (CDKN1A), transcript variant 2, mRNA | -1.162 | 0.056 | -1.319 | 0.016 |
| NM_001946 | dual specificity phosphatase 6 (DUSP6), transcript variant 1, mRNA | -1.321 | 0.076 | -1.295 | 0.078 |
| NM_001402 | eukaryotic translation elongation factor 1 alpha 1 (EEF1A1), mRNA | -1.416 | 0.016 | -1.419 | 0.066 |
| NM_032638 | GATA binding protein 2 (GATA2), mRNA | 1.134 | 0.040 | 1.172 | 0.005 |
| NM_006186 | nuclear receptor subfamily 4, group A, member 2 (NR4A2), transcript variant 1, mRNA | -1.869 | 0.007 | -1.877 | 0.070 |
| NM_002525 | nardilysin (N-arginine dibasic convertase) (NRD1), mRNA | 1.113 | 0.043 | 1.141 | 0.050 |
| NM_002576 | p21/Cdc42/Rac1-activated kinase 1 (STE20 homolog, yeast) (PAK1), mRNA | 1.070 | 0.030 | 1.085 | 0.067 |

## Claims

1. FGF9 for use in a method for alleviating one or more symptoms of anxiety in a mammalian subject, said method comprising acutely administering an effective amount of said FGF9 to said subject.

2. The FGF9 for use according to claim 1, wherein said effective amount of FGF9 is administered to said subject by injection.

3. The FGF9 for use according to claim 1, wherein said subject has been diagnosed with an anxiety disorder.

4. The FGF9 for use according to claim 1, wherein said subject has been diagnosed with major depression disorder.

5. The FGF9 for use according to claim 1, wherein said subject is a human.

6. FGF9 for use in a method for treating an addictive behaviour in a mammalian subject, said method comprising chronically administering an effective amount of said FGF9 to said subject.

7. The FGF9 for use according to claim 6, wherein said effective amount of FGF9 is administered to said subject by injection.

8. The FGF9 for use according to claim 6, wherein said subject has high FGF2 levels.

9. The FGF9 for use according to claim 6, wherein said subject is a human.

10. The FGF9 for use according to claim 1 or 6, wherein said effective amount of FGF9 is administered with a pharmaceutically acceptable carrier.

11. The FGF9 for use according to claim 1 or 6, wherein said FGF9 comprises a recombinant polypeptide.

12. The FGF9 for use according to claim 1 or 6, wherein said effective amount of FGF9 is administered by parenteral or transmucosal administration.

13. The FGF9 for use according to claim 6, wherein said addictive behaviour comprises drinking alcohol, taking drugs, driving fast, or gambling.

## Patentansprüche

1. FGF9 zur Verwendung in einem Verfahren zur Linderung eines Symptoms oder mehrerer Symptome von Angst bei einem Säugetiersubjekt, wobei das Verfahren akutes Verabreichen einer wirksamen Menge des FGF9 an das Säugetier umfasst.

2. FGF9 zur Verwendung nach Anspruch 1, wobei die wirksame Menge an FGF9 durch Injektion an das Säugetier verabreicht wird.

3. FGF9 zur Verwendung nach Anspruch 1, wobei bei dem Säugetiersubjekt eine Angststörung diagnostiziert worden ist.

4. FGF9 zur Verwendung nach Anspruch 1, wobei bei dem Säugetier eine typische Depressionsstörung diagnostiziert wurde.

5. FGF9 zur Verwendung nach Anspruch 1, wobei das Subjekt ein Mensch ist.

6. FGF9 zur Verwendung in einem Verfahren zur Behandlung eines Suchtverhaltens bei einem Säugetiersubjekt, wobei das Verfahren chronisches Verabreichen einer wirksamen Menge des FGF9 an das Subjekt umfasst.

7. FGF9 zur Verwendung nach Anspruch 6, wobei die wirksame Menge an FGF9 dem Subjekt durch Injektion verabreicht wird.

8. FGF9 zur Verwendung nach Anspruch 6, wobei das Subjekt hohe FGF2-Spiegel hat.

9. FGF9 zur Verwendung nach Anspruch 6, wobei das Subjekt ein Mensch ist.

10. FGF9 zur Verwendung nach Anspruch 1 oder 6, wobei die wirksame Menge an FGF9 mit einem pharmazeutisch annehmbaren Träger verabreicht wird.

11. FGF9 zur Verwendung nach Anspruch 1 oder 6, wobei das FGF9 ein rekombinantes Polypeptid umfasst.

12. FGF9 zur Verwendung nach Anspruch 1 oder 6, wobei die wirksame Menge an FGF9 durch parenterale oder transmukosale Verabreichung verabreicht wird.

13. FGF9 zur Verwendung nach Anspruch 6, wobei das Suchtverhalten Trinken von Alkohol, Einnehmen von Drogen, schnelles Fahren oder Spielen umfasst.

## Revendications

1. Facteur de croissance de fibroblastes FGF9 pour utilisation dans un procédé permettant d'atténuer un ou plusieurs symptôme(s) d'anxiété chez un sujet mammifère, lequel procédé comporte le fait d'administrer dudit FGF9 audit sujet, en mode aigu et en quantité efficace.

2. Facteur FGF9 pour utilisation conforme à la revendication 1, étant entendu que c'est par injection qu'on administre ladite quantité efficace de FGF9 audit sujet.

3. Facteur FGF9 pour utilisation conforme à la revendication 1, étant entendu qu'on a diagnostiqué un trouble anxieux chez ledit sujet.

4. Facteur FGF9 pour utilisation conforme à la revendication 1, étant entendu qu'on a diagnostiqué un trouble dépressif majeur chez ledit sujet.

5. Facteur FGF9 pour utilisation conforme à la revendication 1, étant entendu que ledit sujet est un humain.

6. Facteur de croissance de fibroblastes FGF9 pour utilisation dans un procédé permettant de traiter un comportement addictif chez un sujet mammifère, lequel procédé comporte le fait d'administrer dudit FGF9 audit sujet, en mode chronique et en quantité efficace.

7. Facteur FGF9 pour utilisation conforme à la revendication 6, étant entendu que c'est par injection qu'on administre ladite quantité efficace de FGF9 audit sujet.

8. Facteur FGF9 pour utilisation conforme à la revendication 6, étant entendu que ledit sujet présente de hauts niveaux de FGF2.

9. Facteur FGF9 pour utilisation conforme à la revendication 6, étant entendu que ledit sujet est un humain.

10. Facteur FGF9 pour utilisation conforme à la revendication 1 ou 6, étant entendu qu'on administre ladite quantité efficace de FGF9 avec un véhicule pharmacologiquement admissible.

11. Facteur FGF9 pour utilisation conforme à la revendication 1 ou 6, étant entendu que ledit FGF9 comprend un polypeptide recombinant.

12. Facteur FGF9 pour utilisation conforme à la revendication 1 ou 6, étant entendu qu'on administre ladite quantité efficace de FGF9 par voie parentérale ou par voie trans-muqueuse.

13. Facteur FGF9 pour utilisation conforme à la revendication 6, étant entendu que ledit comportement addictif comporte le fait de boire de l'alcool, de consommer des drogues, de conduire vite, ou de s'adonner aux jeux d'argent.
